(19) Europäisches Patentamt
European Patent Office
Office européen des brevets

(11) **EP 3 131 621 B1**

(12) **EUROPÄISCHE PATENTSCHRIFT**

(45) Veröffentlichungstag und Bekanntmachung des Hinweises auf die Patenterteilung:
**28.11.2018 Patentblatt 2018/48**

(51) Int Cl.:
**A61N 1/36** *(2006.01)*      **A61N 1/32** *(2006.01)*
**A61N 1/40** *(2006.01)*

(21) Anmeldenummer: **14720070.3**

(22) Anmeldetag: **16.04.2014**

(86) Internationale Anmeldenummer:
**PCT/EP2014/057734**

(87) Internationale Veröffentlichungsnummer:
**WO 2015/158379 (22.10.2015 Gazette 2015/42)**

(54) **THERAPIEVORRICHTUNG ZUR ERZEUGUNG VON ELEKTROMAGNETISCHEN SCHWINGUNGEN**

THERAPY DEVICE FOR PRODUCING ELECTROMAGNETIC OSCILLATIONS

DISPOSITIF DE THÉRAPIE DESTINÉ À PRODUIRE DES OSCILLATIONS ÉLECTROMAGNÉTIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**22.02.2017 Patentblatt 2017/08**

(73) Patentinhaber: **Baklayan, Alan, E.**
**80331 München (DE)**

(72) Erfinder: **Baklayan, Alan, E.**
**80331 München (DE)**

(74) Vertreter: **Flügel Preissner Schober Seidel Patentanwälte PartG mbB**
**Nymphenburger Strasse 20**
**80335 München (DE)**

(56) Entgegenhaltungen:
**DE-A1-102013 102 275     US-A1- 2006 195 153**
**US-A1- 2007 179 564**

- **Stephan Wiede: "Sanfte Lymphdrainage mit dem Biowave", , 2011, XP055132377, ISBN: 978-1-90-746911-4 Gefunden im Internet: URL:http://www.dermavit-international.net/ ebook_sanfte-lymphdrainage-mit-dem-biowave .pdf [gefunden am 2014-07-30]**
- **ANONYMOUS: "HiToP-Einführung in die Hochtontherapie", GBO MEDIZINTECHNIK AG PROSPEKT, GBO MEDIZINTECHNIK AG, RIMBACH , Mai 2007 (2007-05), Seiten 1-24, XP002545363, Gefunden im Internet: URL:http://www.gbo-medizintechnik.de/de/pd f/Prospekt_Einfuehrung_Hochtontherapie.pdf [gefunden am 2009-09-10]**

EP 3 131 621 B1

**Beschreibung**

Die Erfindung betrifft eine Therapievorrichtung zur Erzeugung von elektromagnetischen Schwingungen.

Aus dem Stand der Technik sind Geräte zur Behandlung von Patienten mit energetischen Schwingungen bekannt. Beispielsweise beschreibt DE 10 2013 102 275 A1 ein solches Behandlungsgerät. Das Gerät erzeugt elektrische Schwingungen, die über ein Kabel an eine Elektrode geleitet werden. Der Patient berührt die Elektrode und die von dem Gerät erzeugten elektrischen Schwingungen werden in den Körper des Patienten geleitet. Die elektrischen Schwingungen treten in Wechselwirkung mit elektrophysiologischen Schwingungen des Körpers. Elektrische Schwingungen treten in jedem Organismus auf. Die von dem Behandlungsgerät erzeugten elektrischen Schwingungen sollen die sich in dem Körper befindenden elektrophysiologischen Schwingungen anregen, verstärken oder unterdrücken, je nachdem, ob die elektrophysiologischen Schwingungen gewünscht oder nicht gewünscht sind. Nicht gewünschte elektrophysiologische Schwingungen können für Krankheiten oder andere Störungen des Organismus verantwortlich sein. Werden elektrophysiologische Schwingungen durch das Behandlungsgerät verstärkt, können positive Effekte der gewünschten elektrophysiologischen Schwingungen unterstützt werden.

Stephan Wiede: "Sanfte Lymphdrainage mit Biowave" befasst sich mit der Behandlung von Patienten mit Wechselspannung, insbesondere zur Lymphdrainage. US 2006/195153 A1 offenbart ein System zum selektiven Stimulieren von unterschiedlichen Körperteilen. Der GBO Medizintechnik AG Prospekt: "HiToP-Einführung in die Hochtontherapie" beschreibt einen Aspekt der Elektrotherapie. US 2007/179564 A1 befasst sich mit der Behandlung von Sehfehlern mittesls elektrischer Energie, Licht- oder Tonenergie.

Der Erfindung liegt die Aufgabe zugrunde, eine Therapievorrichtung bereitzustellen, die eine besonders wirksame Behandlung eines Lebewesens ermöglicht.

Die Aufgabe wird durch die Therapievorrichtung nach Anspruch 1 gelöst. Vorteilhafte Ausgestaltungen sind Gegenstand der abhängigen Ansprüche.

Erfindungsgemäß umfasst die Therapievorrichtung zur Behandlung von Patienten mit elektromagnetischen Schwingungen wenigstens eine Frequenzgeneratoreinheit zur Erzeugung von Wechselspannung und eine Steuereinheit, welche die Frequenzgeneratoreinheit steuert. Die Frequenzgeneratoreinheit ist ausgebildet, die Wechselspannung mit sich zeitlich ändernder Maximalamplitude zu erzeugen, wobei die Maximalamplitude die Differenz zwischen maximaler Amplitude und minimaler Amplitude einer Periode der Wechselspannung ist. Die Steuereinheit variiert die Maximalamplitude wiederholt zwischen einer unteren Amplitudenschwelle und einer oberen Amplitudenschwelle. Die Steuereinheit steuert die Frequenzgeneratoreinheit derart, dass die Maximalamplitude sich über einen ersten Zeitraum von einer Startamplitude zu einer Zielamplitude ändert und anschließend über einen zweiten Zeitraum auf die Startamplitude zurückkehrt, wobei der erste Zeitraum mindestens 10-fach größer als der zweite Zeitraum ist.

Die von der Therapievorrichtung erzeugten elektromagnetischen Schwingungen dienen vorzugsweise der Behandlung von Menschen oder Tieren mit den von dem Frequenzgenerator erzeugten elektrischen Schwingungen. Wie zuvor dargestellt, lassen sich mit den elektromagnetischen Schwingungen gewünschte elektrophysiologische Effekte im Körper des menschlichen oder tierischen Patienten verstärken oder abschwächen. Dazu berührt der Patient vorzugsweise eine Elektrode, die über ein Kabel mit der Frequenzgeneratoreinheit verbunden ist. Der Patient kann die Elektrode halten oder die Elektrode kann an dem Patienten mittels eines Fixierbands oder Ähnlichem angebracht sein.

Die Frequenzgeneratoreinheit weist vorzugsweise einen Frequenzgenerator auf, der sich durch eine Steuereinheit derart steuern lässt, dass die von der Frequenzgeneratoreinheit ausgegebene Wechselspannung variiert werden kann. Die Steuereinheit kann ein Prozessor, ein Rechner oder eine analoge Steuereinheit sein. Die Maximalamplitude, oft auch als Spitze-Spitze-Spannung bezeichnet, gibt die Größe der Amplitude der Wechselspannung an. Die Maximalamplitude bezieht sich dabei auf die minimale und maximale Spannung, die während einer Periode der Wechselspannung vorliegt. Insbesondere entspricht die Amplitude der Wechselspannung der Spannung der Wechselspannung.

Die Steuereinheit variiert die Maximalamplitude der Wechselspannung derart, dass die Maximalamplitude zwischen einem minimalen Wert, der unteren Amplitudenschwelle, und einem maximalen Wert, der oberen Amplitudenschwelle, wiederholt verändert wird. Dies ergibt dann einen Verlauf der Wechselspannung, der sich durch eine Grundwechselspannung und eine einhüllende Spannung beschreiben lässt. Die Grundwechselspannung hat die Frequenz der Wechselspannung, während die einhüllende Spannung sich durch eine Funktion beschreiben lässt, die sich zwischen einem unteren Grenzwert und einem oberen Grenzwert verändert. Mathematisch lässt sich dies als ein Produkt der Amplitude der Grundwechselspannung mit der Funktion der einhüllenden Spannung beschreiben. Die Funktion der einhüllenden Spannung nimmt somit wiederholt die Werte des unteren Grenzwerts und des oberen Grenzwerts an. Beispielsweise nimmt die Funktion der einhüllenden Spannung Werte zwischen 0 und 1 an. Das Produkt aus der maximalen Amplitude der Grundwechselspannung sowie dem unteren Grenzwert oder dem oberen Grenzwert ergibt die untere Amplitudenschwelle und die obere Amplitudenschwelle.

Die untere Amplitudenschwelle und die obere Amplitudenschwelle können Werte zwischen 0 V bis 20 V, insbesondere zwischen 0 V und 16 V, annehmen. Insbesondere ist die Maximalamplitude der Grundwechselspannung zwischen 0,1 V und 20 V, insbesondere zwischen 0,1 V und 16 V, während die Spannung der einhüllenden Funktion Werte zwischen 0 V und 1 V annimmt. In einer bevorzugten Ausführungsform nimmt die Wechselspannung absolute Werte zwischen -8 V und 16 V an; absolute Werte sind insbesondere die Werte, welche an der Frequenzgeneratoreinheit, beispielsweise mit Hilfe eines Oszilloskops, gemessen werden können.

Vorzugsweise ist die untere Amplitudenschwelle der Wechselspannung gleich null, so dass die Amplituden der Wechselspannung zwischen Zeitspannen mit keiner Spannung und Zeitspannen mit der Maximalamplitude variiert werden. Alternativ ist die untere Amplitudenschwelle der Wechselspannung größer als null; insbesondere ist der untere Grenzwert der einhüllenden Funktion größer als null.

Bevorzugt werden die untere Amplitudenschwelle und die obere Amplitudenschwelle im Verlauf der Anwendung der Therapievorrichtung in einer Vielzahl von Wiederholungen eingenommen. Die Anzahl kann beispielsweise von der Frequenz der Grundwechselspannung abhängen.

Es hat sich gezeigt, dass die Variation der Wechselspannung zwischen einer unteren Amplitudenschwelle und einer oberen Amplitudenschwelle besonders gute elektrophysiologische Effekte im Körper des Patienten hervorruft.

Es ist bevorzugt, dass die Maximalamplitude periodisch variiert wird. Insbesondere steuert die Steuereinheit die Maximalamplitude der Wechselspannung periodisch. Alternativ lässt sich dies so darstellen, dass die Wechselspannung mit einer einhüllenden Funktion variiert wird, die sich periodisch zwischen dem unteren Grenzwert und dem oberen Grenzwert bewegt. Insbesondere ist die einhüllende Funktion eine periodische Funktion. Beispielweise ist die Gesamtwechselspannung somit ein Produkt der Grundwechselspannung mit einer periodischen einhüllenden Wechselspannung. Es hat sich gezeigt, dass eine periodische Variation oder Modulation der Wechselspannung eine besonders starke Wirkung im Körper des Patienten hervorruft.

Es ist bevorzugt, dass die untere Amplitudenschwelle und die obere Amplitudenschwelle jeweils innerhalb einer Periode der Wechselspannung zeitlich konstant sind.

Vorzugsweise sind die untere Amplitudenschwelle oder die obere Amplitudenschwelle über mehrere Perioden der Wechselspannung zeitlich konstant. Die einhüllende Funktion lässt sich somit vorzugsweise als eine, insbesondere periodische, Rechteckspannung beschreiben. Die Maximalamplitude nimmt insbesondere die Werte der unteren und der oberen Amplitudenschwelle an, die dem Produkt der Grenzwerte der einhüllenden Wechselspannung und der maximalen Amplitude der Grundwechselspannung entspricht. Alternativ kann die Grundwechselspannung mit einer sinusförmigen Funktion als einhüllende Funktion variiert werden.

Es ist bevorzugt, dass das Frequenzverhältnis der Frequenz der Wechselspannung zu der Frequenz der Variation der Maximalamplitude eine ganzzahlige gerade Zahl ist. In dieser bevorzugten Ausführungsform ist die Frequenz der Grundwechselspannung gleich einem ganzzahligen Vielfachen der Frequenz der einhüllenden Spannung, oder anders ausgedrückt: Frequenz (Grundwechselspannung) = $2^x$ Frequenz (einhüllende Wechselspannung), wobei x eine natürliche Zahl ist. Dieses bevorzugte Verhältnis bedeutet, dass die Frequenz der Grundwechselspannung und die Frequenz der einhüllenden Wechselspannung sich wie die Oktaven einer Tonleiter verhalten. Es hat sich gezeigt, dass dieses Verhältnis besonders gute elektrophysiologische Effekte im Körper des Patienten hervorruft.

Es ist bevorzugt, dass das Frequenzverhältnis zwischen 2 und 65536, insbesondere zwischen 2 und 256, beträgt. Weiter vorzugsweise ist das Frequenzverhältnis zwischen der Frequenz der Grundwechselspannung und der Frequenz der einhüllenden Wechselspannung 128 beziehungsweise sieben Oktaven. Es hat sich gezeigt, dass der Körper des Patienten besonders gut auf die oben angegebenen Frequenzverhältnisse reagiert.

Es ist bevorzugt, dass die Wechselspannung eine Rechteckspannung ist. Die sich in einer bevorzugten Ausführungsform ergebende Gesamtspannung setzt sich aus einer Grundwechselspannung in Rechteckform und einer einhüllenden Wechselspannung in Rechteckform zusammen. Die Rechteckwechselspannung der Grundwechselspannung und/oder der einhüllenden Wechselspannung kann durch Summation von sinusförmigen Spannungen erzeugt werden, wie dies aus dem Stand der Technik bekannt ist. Dies hat zur Folge, dass besonders viele Wechselspannungen unterschiedlicher Frequenzen in dem Körper des Patienten wirken, so dass ein besonders hoher physiologischer Effekt erzielt werden kann.

Bevorzugt ist die Frequenzgeneratoreinheit ausgebildet, die Wechselspannung mit sich zeitlich ändernder Frequenz zu erzeugen. Die Steuereinheit variiert die Frequenz der von der Frequenzgeneratoreinheit ausgegebenen Wechselspannung gemäß einer stetigen Funktion von einer unteren Grenzfrequenz zu einer oberen Grenzfrequenz oder von der oberen Grenzfrequenz zu der unteren Grenzfrequenz.

Vorzugsweise weist die Therapievorrichtung gemäß dem weiteren Aspekt der Erfindung auch die Merkmale und Vorteile der zuvor beschriebenen Therapievorrichtung auf.

Die Frequenz der Wechselspannung wird jeweils vorzugsweise entweder von der unteren Grenzfrequenz zu der oberen Grenzfrequenz oder von der oberen Grenzfrequenz zu der unteren Grenzfrequenz variiert, jedoch nicht sowohl von der unteren Grenzfrequenz zu der oberen Grenzfrequenz und von der oberen Grenzfrequenz zu der unteren Grenzfrequenz. Diese auch als einseitig bezeichnete Variation der Frequenz der Wechselspannung ist auch als Wobbeln bekannt und hat, wie durch die Erfindung herausgefunden wurde, einen besonders hohen elektrophysiologischen Effekt. Die Variation der Frequenz kann beispielsweise mittels eines Wobbelgenerators erzeugt werden.

Die Frequenzvariation folgt vorzugsweise dem Verlauf einer stetigen Funktion zwischen der unteren Grenzfrequenz und der oberen Grenzfrequenz. Eine steige Funktion ist im Sinne dieser Offenbarung eine Funktion, die im mathematischen Sinne stetig ist. Die Stetigkeit ist beim Sprung zwischen der oberen Grenzfrequenz und der unteren Grenzfrequenz nicht gegeben. Vorzugsweise ist die stetige Funktion eine lineare Funktion, so dass die Frequenz linear zwischen der oberen Grenzfrequenz und der unteren Grenzfrequenz geändert wird.

Es ist bevorzugt, dass die Steuereinheit die Frequenz der von der Frequenzgeneratoreinheit ausgegebenen Wechselspannung periodisch zwischen der unteren Grenzfrequenz und der oberen Grenzfrequenz variiert. Dies bedeutet, dass in dieser bevorzugten Ausführungsform die Frequenz der Wechselspannung periodisch entweder von der unteren Grenzfrequenz auf die obere Grenzfrequenz erhöht oder alternativ von der oberen Grenzfrequenz zu der unteren Grenzfrequenz erniedrigt wird. Dadurch wird eine wiederholte Variation der Frequenz der Wechselspannung bereitgestellt, die einen besonders guten elektrophysiologischen Effekt bereitstellt. Vorzugsweise springt die Frequenz nach Erreichen der oberen Grenzfrequenz auf die untere Grenzfrequenz zurück, ohne die dazwischen liegenden Frequenzen abzurufen; alternativ springt die Frequenz nach Erreichen der unteren Grenzfrequenz auf die obere Grenzfrequenz zurück, ohne die dazwischen liegenden Frequenzen abzurufen.

Es ist bevorzugt, dass die Therapievorrichtung eine zweite Frequenzgeneratoreinheit zur Erzeugung von Wechselspannung und/oder eine dritte Frequenzgeneratoreinheit zur Erzeugung von Wechselspannung aufweist. Die Steuereinheit steuert die zweite Frequenzgeneratoreinheit und/oder die dritte Frequenzgeneratoreinheit. Vorzugsweise sind die zweite Frequenzgeneratoreinheit und die dritte Frequenzgeneratoreinheit gegeneinander und/oder gegenüber der ersten Frequenzgeneratoreinheit galvanisch getrennt.

Die zweite und/oder dritte Frequenzgeneratoreinheit sind insbesondere analog zu der ersten Frequenzgeneratoreinheit ausgebildet. Zudem kann auch eine vierte, fünfte oder mehr Frequenzgeneratoreinheiten vorgesehen werden die bevorzugt ebenfalls wie die erste Frequenzgeneratoreinheit aufgebaut sind. Die Steuereinheit steuert jeweils die vorgesehenen Frequenzgeneratoreinheiten.

Bevorzugt sind die Frequenzgeneratoreinheiten voneinander galvanisch (elektrisch) getrennt, so dass sich die Frequenzgeneratoreinheiten nicht gegenseitig beeinflussen können. Eine gegenseitige elektrische Störung, die ungewünschte Interferenzen zur Folge haben kann, lässt sich somit vermeiden. Ferner wird bevorzugt auch vermieden, dass Ströme zwischen den einzelnen Frequenzgeneratoreinheiten fließen. Vorzugsweise sind die einzelnen Frequenzgeneratoreinheiten zusätzlich gegen elektromagnetische Strahlung abgeschirmt.

Bevorzugt umfasst eine Therapievorrichtung zur Erzeugung von elektromagnetischen Schwingungen eine erste Frequenzgeneratoreinheit zur Erzeugung von Wechselspannung mit einer ersten Phase, eine zweite Frequenzgeneratoreinheit zur Erzeugung von Wechselspannung mit einer zweiten Phase und eine Steuereinheit, welche die erste Frequenzgeneratoreinheit und die zweite Frequenzgeneratoreinheit steuert. Die Steuereinheit regelt die Phasenverschiebung zwischen der ersten Phase und der zweiten Phase derart, dass sie zwischen 110° und 130°, insbesondere 120°, beträgt.

Vorzugsweise weist die Therapievorrichtung gemäß dem weiteren Aspekt der Erfindung auch die Merkmale und Vorteile die zuvor beschriebenen Therapievorrichtungen auf.

Bevorzugt sind die einzelnen Frequenzgeneratoreinheiten jeweils mit einer Elektrode verbunden. Alternativ können die Frequenzgeneratoreinheiten mit einer gemeinsamen Elektrode verbunden sein. Die Elektrode ist mit dem Patienten in direktem Kontakt. Die erzeugte Wechselspannung kann eine Rechteckspannung oder eine sinusförmige Spannung sein. Es hat sich herausgestellt, dass die Phasenverschiebung der von der ersten und zweiten Frequenzgeneratoreinheit ausgegebenen Wechselspannung einen besonders guten elektrophysiologischen Effekt hervorruft.

Es ist bevorzugt, dass die Therapievorrichtung ferner eine dritte Frequenzgeneratoreinheit zur Erzeugung von Wechselspannung mit einer dritten Phase aufweist. Die Steuereinheit regelt die Phasenverschiebung zwischen der zweiten Phase und der dritten Phase derart, dass diese zwischen 110° und 130°, insbesondere 120°, beträgt.

Die dritte Frequenzgeneratoreinheit kann mit einer eigenen Elektrode oder mit der Elektrode verbunden sein, die auch mit der zweiten und ersten Frequenzgeneratoreinheit verbunden ist. Bevorzugt ergibt sich demnach, dass die einzelnen Phasen der von den Frequenzgeneratoreinheiten erzeugten Wechselspannungen zwischen 110° und 130° phasenverschoben sind. Durch das Vorsehen einer dritten Wechselspannung kann der elektrophysiologische Effekt bei dem Patienten weiter erhöht werden.

Es ist bevorzugt, dass die die Steuereinheit die Frequenzgeneratoreinheiten derart regelt, dass die Maximalamplitude der von der ersten Frequenzgeneratoreinheit erzeugten Wechselspannung größer als die Maximalamplitude der von der zweiten Frequenzgeneratoreinheit erzeugten Wechselspannung ist und dass vorzugsweise die Maximalamplitude der von der zweiten Frequenzgeneratoreinheit erzeugten Wechselspannung größer als die Maximalamplitude der von der dritten Frequenzgeneratoreinheit erzeugten Wechselspannung ist.

Die Maximalamplitude ist so, wie sie oben beschrieben wurde, definiert. Insbesondere ist die von der ersten Frequenzgeneratoreinheit erzeugte Wechselspannung doppelt so groß wie die von der zweiten Frequenzgeneratoreinheit erzeugte Wechselspannung. Weiter vorzugsweise ist die von der zweiten Frequenzgeneratoreinheit erzeugte Wechselspannung doppelt so groß wie die von der dritten Frequenzgeneratoreinheit erzeugte Wechselspannung. Es ergibt sich so ein bevorzugtes Amplitudenverhältnis zwischen den einzelnen Wechselspannungen von 4:2:1. Die abfallende Größe der Amplituden der einzelnen Wechselspannungen wird auch als Kaskadierung bezeichnet. Somit ergibt sich vorzugsweise zusammen mit der Phasenverschiebung eine an dem Patienten anliegende Spannungsabfolge, die sich jeweils halbiert und um 110° bis 130° phasenverschoben ist. Diese anliegende Spannung kann die elektrophysiologische Wirkung der Therapievorrichtung weiter erhöhen.

Es ist bevorzugt, dass die zweite Frequenzgeneratoreinheit und die dritte Frequenzgeneratoreinheit gegeneinander und/oder gegenüber der ersten Frequenzgeneratoreinheit galvanisch getrennt sind. Es gelten insbesondere die zuvor dargelegten Überlegungen hinsichtlich dieses Merkmals. Selbst wenn der Patient zwei der drei Elektroden berührt, kann kein Strom zwischen den beiden Elektroden fließen.

Es ist bevorzugt, dass die erste Frequenzgeneratoreinheit einen ersten Frequenzausgang, an dem die erzeugte Wechselspannung anliegt, und einen Neutralausgang aufweist und/oder dass die zweite Frequenzgeneratoreinheit einen zweiten Frequenzausgang, an dem die erzeugte Wechselspannung anliegt, und einen zweiten Neutralausgang aufweist und/oder dass die dritte Frequenzgeneratoreinheit einen dritten Frequenzausgang, an dem die erzeugte Wechselspannung anliegt, und einen dritten Neutralausgang aufweist.

Es ist bevorzugt, dass die Therapievorrichtung ferner einen ersten Elektrodenausgang zum Anschließen einer ersten Elektrode, der mit der ersten Frequenzausgang verbunden ist, und/oder einen zweiten Elektrodenausgang zum Anschließen einer zweiten Elektrode, der mit der zweiten Frequenzausgang verbunden ist, und/oder einen dritten Elektrodenausgang zum Anschließen einer dritten Elektrode, der mit der dritten Frequenzausgang verbunden ist, aufweist. Ein vorzugsweise aus Metall, insbesondere aus Edelmetall, hergestellter Behälter zur Aufnahme einer Probe ist zwischen dem ersten Elektrodenausgang und dem ersten Frequenzausgang und/oder zwischen dem zweiten Elektrodenausgang und dem zweiten Frequenzausgang und/oder zwischen dem dritten Elektrodenausgang und dem dritten Frequenzausgang elektrisch geschaltet.

Der Patient berührt insbesondere die erste Elektrode, die über ein Kabel mit dem ersten Elektrodenausgang verbunden ist. Vorzugsweise berührt der Patient auch die zweite Elektrode, die über ein Kabel mit dem zweiten Elektrodenausgang verbunden ist. Weiter vorzugsweise berührt der Patient zudem die dritte Elektrode, die über ein Kabel mit dem dritten Elektrodenausgang verbunden ist. Die zweite Elektrode und/oder die dritte Elektrode können wie die erste Elektrode ausgestaltet sein. Die erste und/oder die zweite Elektrode und/oder die dritte Elektrode können auch als eine Antenne zur Aussendung von elektromagnetischen Wellen ausgebildet sein.

Die Frequenzgeneratoreinheiten erzeugen vorzugsweise jeweils eine Wechselspannung, die an dem entsprechenden Frequenzausgang anliegt. Über die Elektrodenausgänge werden die jeweiligen Wechselspannungen an die entsprechenden Elektroden übertragen. Die Spannung kann vorzugsweise zwischen 0,1 V und 20 V, vorzugsweise zwischen 0,1 V und 16 V, variiert werden.

Vorzugsweise bilden der Neutralausgang und der Frequenzausgang der jeweiligen Frequenzgeneratoreinheit zwei Pole für die von der Frequenzgeneratoreinheit erzeugte Wechselspannung, so dass zwischen dem Frequenzausgang und dem Neutralausgang die erzeugte Wechselspannung gemessen werden kann. Beispielsweise kann die Wechselspannung an den Patient angelegt werden, indem dieser eine mit dem Frequenzausgang verbundene Elektrode und eine mit dem Neutralausgang verbundene Elektrode berührt. Jedoch ist es alternativ möglich, dass der Patient nur eine einzige, mit dem Frequenzausgang verbundene Elektrode berührt.

In einer alternativen Ausführungsform kann der Neutralausgang geerdet sein, beispielsweise durch Anschluss der Therapievorrichtung an eine externe Erdung. Bevorzugt kann der Frequenzausgang mit einer Offsetspannung belegt sein, so dass die Wechselspannung beispielsweise nur mit einer positiven Spannung oder einer negativen Spannung oszilliert. Die Frequenzgeneratoreinheit hat vorzugsweise einen Innenwiderstand von 100 Ω bis 100 kΩ, insbesondere von 1 kΩ.

Der eine Elektrode in Form eines Bechers darstellende Behälter dient bevorzugt der Aufnahme von Proben, die die von der Frequenzgeneratoreinheit erzeugte Wechselspannung auf eine für die Behandlung des Patienten positive Weise beeinflussen soll. Eine Elektrode in Becherform dient zur Aufnahme von Proben, um vorzugsweise deren energetischen Informationen in die therapeutische Anwendung wie zum Beispiel die Wechselspannung mit einfließen zu lassen. Dies geschieht beispielsweise durch eine Veränderung der Kapazität des Behälters. Die geänderte Kapazität verändert dann die anliegende Wechselspannung. Die Proben können Eigenproben des Patienten, wie beispielsweise Blut oder Speichel, sein oder belastende Substanzen, wie beispielsweise Allergene, Schwermetalle, Viren oder Bakterien, enthalten.

Es ist bevorzugt, dass die Steuereinheit die zweite Frequenzgeneratoreinheit und/oder die dritte Frequenzgeneratoreinheit derart steuert, dass die Maximalamplitude der Wechselspannung über einen ersten Zeitraum von einer Startamplitude zu einer Zielamplitude variiert wird und anschließend die Maximalamplitude der Wechselspannung über einen zweiten Zeitraum auf die Startamplitude zurückkehrt, wobei der erste Zeitraum größer, insbesondere mindestens 10-fach oder 100-fach, als der zweite Zeitraum ist.

Dadurch, dass der zweite Zeitraum kleiner als der erste Zeitraum ist, wird die Maximalamplitude der Wechselspannung im ersten Zeitraum langsam verändert, während die Maximalamplitude während des zweiten Zeitraums schnell verändert wird. Der Verlauf der Änderung der Maximalamplitude kann beispielsweise ähnlich dem einer Dreiecksspannung sein. Der zweite Zeitraum beträgt vorzugsweise zwischen 0,1 ms bis 1 s. Der erste Zeitraum erstreckt sich vorzugsweise von einer Sekunde bis zu 100 Sekunden. Diese Variation der Maximalamplitude wird auch als einseitiger Sweep bezeichnet.

Vorzugsweise ändert sich die Maximalamplitude der Wechselspannung dem Verlauf einer stetigen Funktion, insbesondere einer linearen Funktion, folgend von der Startamplitude zu der Zielamplitude. Die Rückkehr der Maximalamplitude der Wechselspannung von der Zielamplitude zu der Startamplitude kann ein Sprung sein und muss daher nicht einer stetigen Funktion folgend ausgebildet sein. Alternativ kann die Rückkehr von der Maximalamplitude der Wechselspannung von der Zielamplitude zu der Startamplitude ähnlich wie die Änderung der Maximalamplitude von der Startamplitude zu der Zielamplitude sein, jedoch über einen kürzeren Zeitraum.

Die Startamplitude der Maximalamplitude kann beispielsweise zwischen 0 V und 1 V, insbesondere 0,1 V gewählt werden. Die Zielamplitude der Maximalamplitude kann zwischen 1 V und 16 V gewählt werden, so dass während der Variation der Amplitude ein großer Bereich abgefahren werden kann. Diese Ausführungsform entspricht eine kontinuierlichen Erhöhung der Maximalamplitude dar. Alternativ kann auch ein Bereich von 1 V bis 16 V als Startamplitude und ein Bereich von 0 V bis 1 V, insbesondere 0,1 V, als Zielamplitude vorgesehen sein, was einer kontinuierlichen Reduktion der Maximalamplitude entspricht. In einer weiteren Ausführungsform kann der erste Zeitraum und der zweite Zeitraum etwa gleich groß gewählt werden, was einem zweiseitigen Sweep entspricht. Die Variation der Maximalamplitude kann beispielsweise helfen, die beste Maximalamplitude zur Behandlung des Patienten zu bestimmen.

Es ist bevorzugt, dass der erste Elektrodenausgang wenigstens zwei Anschlüsse, insbesondere drei Anschlüsse umfasst, wobei vorzugsweise der zweite Elektrodenausgang einen Anschluss umfasst, der mit einem der Anschlüsse des ersten Elektrodenausgangs eine vorzugsweise koaxiale Steckverbindung, insbesondere einen BNC-Anschluss, bildet.

Es ist somit bevorzugt, dass die Therapievorrichtung zwei Anschlüsse, insbesondere Steckanschlüsse (zum Beispiel für Bananenstecker), aufweist, an denen die von dem Frequenzgenerator am Elektrodenausgang erzeugte Wechselspannung abgegriffen werden kann. Ferner hat die Therapievorrichtung vorzugsweise eine koaxiale Steckverbindung, bei der insbesondere der Innenleiter mit dem Frequenzausgang des Frequenzgenerators und weiter vorzugsweise der Außenleiter mit dem Neutralausgang des Frequenzgenerators verbunden ist. An der Steckverbindung können daher zwei Elektroden angeschlossen werden. Es ist auch möglich, dass an der Steckverbindung lediglich der Neutralausgang der Frequenzgeneratoreinheit angeschlossen wird und die Spannung mittels der beiden anderen Anschlüsse des ersten Ausgangs verbunden ist. An der Therapievorrichtung ist vorzugsweise der Stecker der Steckverbindung vorgesehen, wohingegen die Elektroden mit der Buchse der Steckverbindung verbunden sind. Eine umgekehrte Anordnung ist auch möglich. Die Steckverbindung kann ein BNC-Anschluss, ein SMA-Anschluss oder eine andere Steckverbindung sein, die insbesondere für Hochfrequenzanschlüsse geeignet ist.

Es ist bevorzugt, dass die Elemente der Frequenzgeneratoreinheit innerhalb eines Gehäuses eingebaut sind, wobei die Anschlüsse von außen zugänglich sind. Insbesondere ist auch der Behälter im Gehäuse angebracht, so dass die Proben in den Behälter eingebracht werden können, ohne das Gehäuse zu öffnen. Der Behälter kann vorzugsweise mittels eines Deckels verschlossen werden und ist zweckmäßigerweise auswechselbar.

Bevorzugt weist die Therapievorrichtung einen Energiespeicher, insbesondere einen Akkumulator auf, der die Frequenzgeneratoreinheit und/oder die Steuereinheit mit elektrischer Energie versorgt.

Es ist somit bevorzugt möglich, dass die Therapievorrichtung von einem Stromnetz getrennt betrieben werden kann. Zum einen ermöglicht dies, dass die durch das Stromnetz erzeugte elektromagnetische Schwingung, wie zum Beispiel die Strahlung der 50Hz-Netzfrequenz, vermieden werden kann. Insbesondere die Vermeidung von elektromagnetischen Schwingungen in der Nähe des Patienten hat den positiven Effekt, dass keine externen Schwingungen die Behandlung des Patienten beeinflussen.

Der Energiespeicher kann insbesondere eine wiederaufladbare Batterie sein, die mittels eines Ladegeräts, das an die Therapievorrichtung anschließbar ist, aufgeladen werden kann. Alternativ kann das Ladegerät in der Therapievorrichtung eingebaut sein, wobei das Ladegerät entweder über ein externes Netzteil (beispielsweise mit 18V-Ausgangsspannung) oder direkt über die Netzspannung betrieben werden kann. Das Vorsehen eines Energiespeichers hat den Vorteil, dass die Therapievorrichtung variabler einsetzbar ist, da sie nicht an das Vorhandensein eines elektrischen Stromnetzes geknüpft ist. Der Energiespeicher ist vorzugsweise auch innerhalb des Gehäuses der Therapievorrichtung angeordnet.

Es ist bevorzugt, dass die Frequenzgeneratoreinheit zur Erzeugung von Wechselspannung mit unterschiedlichen Frequenzen, insbesondere zwischen 0,1 Hz und 100 MHz, mehr insbesondere zwischen 0,1 Hz und 20 MHz, ausgebildet ist, wobei vorzugsweise die Frequenzgeneratoreinheit ausgebildet ist, die Spannung und die Art der ausgegebenen Wechselspannung zu verändern.

Die Abdeckung eines großen Frequenzbereichs zwischen 0,1 Hz und 100 MHz, insbesondere zwischen 1 Hz und 20 MHz, hat den Vorteil, dass die Therapievorrichtung in verschiedensten Anwendungsbereichen verwendet werden kann. Die Maxiamalspannung, die Spitzen-Spitzen-Spannung, die Amplitudenspannung oder die Effektivspannung können vorzugsweise zwischen 0,1 V und 16 V variiert werden. Vorzugsweise kann die Frequenzgeneratoreinheit eine Wechselspannung mit sinuswellenförmigem, rechteckigem oder dreieckigem Wellenprofil erzeugen. Die einzelnen Wellenprofile haben erfahrungsgemäß jeweils besondere Vorteile bei den jeweiligen Behandlungen.

Es ist bevorzugt, dass die Frequenz, die Spannung und/oder die Art der ausgegebenen Wechselspannung mittels der Steuereinheit steuerbar sind.

Vorzugsweise ist die Steuereinheit innerhalb des Gehäuses der Therapievorrichtung angeordnet und insbesondere durch einen Mikroprozessor realisiert. Weiter vorzugsweise ist der Energiespeicher mit der Steuereinheit verbunden, so dass der Frequenzgenerator über die Steuereinheit mit Energie versorgt wird. Die Steuereinheit gibt vorzugsweise die Frequenz, die Spannung und/oder die Art der ausgegebenen Wechselspannung an den Frequenzgenerator aus, der wiederum eine Wechselspannung mit den von der Steuereinheit angegebenen Parametern ausgibt.

Es ist bevorzugt, dass die Therapievorrichtung eine Anzeigeeinrichtung und/oder eine Eingabeeinrichtung aufweist. Die Anzeigeeinrichtung zeigt die Frequenz, die Spannung und/oder die Art der ausgegebenen Wechselspannung an, wobei vorzugsweise die Frequenz, die Spannung und/oder die Art der ausgegebenen Wechselspannung mittels der Eingabeeinrichtung einstellbar sind.

Die Eingabeeinrichtung ist vorzugsweise mit der Steuereinheit verbunden. Ferner kann die Steuereinheit mit der Anzeigeeinrichtung, die beispielsweise ein Display sein kann, verbunden sein und diese ansteuern. Die Anzeigeeinrichtung und die Eingabeeinrichtung sind vorzugsweise an dem Gehäuse der Therapievorrichtung vorgesehen. Die Art der Wechselspannung kann beispielsweise durch einen Begriff oder durch eine visuelle Darstellung der Wechselspannung angezeigt werden. Die Frequenz und/oder die Spannung kann mittels einer numerischen Eingabe eingegeben werden. Es ist aber auch möglich, dass die Steuereinheit einen Speicher aufweist, in dem verschiedenste Frequenzen eingespeichert sind, die jeweils mit einer Nummer versehen sind. Durch Eingabe einer Nummer an der Eingabeeinrichtung kann die gewünschte Frequenz eingegeben werden.

Alternativ oder weiter bevorzugt ist der Frequenzgenerator zur Erzeugung von wenigstens zwölf festgelegten Frequenzen ausgebildet. Die Frequenzen liegen in einem Frequenzbereich, der eine untere Grenzfrequenz und eine obere Grenzfrequenz aufweist. Die obere Grenzfrequenz ist doppelt so groß wie die untere Grenzfrequenz, wobei vorzugsweise die obere Grenzfrequenz nicht Teil des Frequenzbereichs ist.

Bevorzugt ist der Frequenzgenerator ausgebildet, zumindest zwölf festgelegte, unterschiedliche erste Frequenzen zu erzeugen. Diese zwölf Frequenzen liegen alle in innerhalb eines ersten Bereichs, dessen Grenzen bevorzugt einem Oktavengesetz folgen. So ist die untere Grenzfrequenz gleich der niedrigsten Frequenz der zwölf ersten Frequenzen. Die obere Grenzfrequenz ist durch die doppelte Frequenz der unteren Grenzfrequenz gebildet, so dass sich ein oktavenspezifisches Verhältnis von 2:1 ergibt. Bevorzugt ist die obere Grenzfrequenz aus dem ersten Bereich ausgeschlossen.

Es ist bevorzugt, dass das Verhältnis der Frequenzen zur unteren Grenzfrequenz 1, 14:13, 9:8, 6:5, 5:4, 4:3, 7:5, 3:2, 8:5, 5:3, 9:5 oder 13:7 beträgt. Ein Verhältnis der Frequenz zur unteren Grenzfrequenz von 2:1 entspricht der ersten Oberschwingung der unteren Grenzfrequenz und kann auch als Frequenz ausgewählt werden. Diese Frequenzverhältnisse entsprechen der Harmonikalischen Lehre und sind besonders geeignet, die Meridiane des Körpers anzuregen. Alternativ kann ein weiteres Frequenzverhältnis gewählt werden. Bei diesem unterscheiden sich benachbarte Frequenzen der zwölf Frequenzen, also die erste zu der zweiten, die zweite zu der dritten und in diesem Sinne weiter, um ein konstantes Verhältnis $\sqrt[12]{2}$. Auch hier ergibt sich ein Verhältnis von Grundfrequenz zu erster Oberschwingung von 2:1.

Ferner wird hierin ein Verfahren zur Steuerung einer Therapievorrichtung beschrieben, die eine Frequenzgeneratoreinheit zur Erzeugung von Wechselspannung umfasst. Das Verfahren umfasst den Schritt Variieren der Maximalamplitude der Wechselspannung wiederholt zwischen einer unteren Amplitudenschwelle und einer oberen Amplitudenschwelle, wobei die Maximalamplitude die Differenz zwischen maximaler Amplitude und minimaler Amplitude einer Periode der Wechselspannung ist.

Insbesondere wird die Maximalamplitude periodisch variiert. Ferner gelten bevorzugt die im Hinblick auf die Therapievorrichtung dargelegten bevorzugten Merkmale und Vorteile.

Ferner wird hier ein Verfahren zur Steuerung einer Therapievorrichtung zur Erzeugung von elektromagnetischen Schwingungen bereitgestellt, wobei die Therapievorrichtung eine erste Frequenzgeneratoreinheit zur Erzeugung von Wechselspannung mit einer ersten Phase und eine zweiter Frequenzgeneratoreinheit zur Erzeugung von Wechselspannung mit einer zweiten Phase umfasst. Das Verfahren umfasst den Schritt des Regelns der ersten Phase in Bezug auf die zweite Phase derart, dass die Phasenverschiebung zwischen 110° und 130°, insbesondere 120°, beträgt.

Bevorzugt gelten die im Hinblick auf die Therapievorrichtung dargelegten bevorzugten Merkmale und Vorteile.

Ferner wird hier ein weiteres Verfahren zum Steuern einer Therapievorrichtung zur Erzeugung von elektromagnetischen Schwingungen angegeben, bei dem die Therapievorrichtung eine Frequenzgeneratoreinheit zur Erzeugung von Wechselspannung umfasst. Das Verfahren umfasst den Schritt des Variierens der Frequenz der von der Frequenzgeneratoreinheit ausgegebenen Wechselspannung gemäß einer stetigen Funktion, insbesondere gemäß einer linearen Funktion, von einer unteren Grenzfrequenz zu einer oberen Grenzfrequenz oder von der oberen Grenzfrequenz zu der unteren Grenzfrequenz. Bevorzugt gelten die im Hinblick auf die Therapievorrichtung dargelegten bevorzugten Merkmale und Vorteile.

Im Folgenden wird die Erfindung anhand eines Ausführungsbeispiels in Verbindung mit den Zeichnungen beschrieben. Dabei zeigt:

Fig. 1        eine schematische Darstellung einer Therapievorrichtung;

Fig. 2        eine schematische Darstellung der Therapievorrichtung bei Behandlung eines Patienten;

Fig. 3        eine perspektivische Ansicht der Therapievorrichtung;

Fig. 4        eine weitere perspektivische Ansicht der Therapievorrichtung;

Fig. 5        eine Ansicht einer Anzeigeeinrichtung der Therapievorrichtung;

Fig. 6a, b und c    eine Tabelle der in einer Steuereinheit der Therapievorrichtung gespeicherten Frequenzen,

Fig. 7        den Amplitudenverlauf der von Therapievorrichtung erzeugten Wechselspannung;

Fig. 8        die Phasenbeziehung der von der Therapievorrichtung erzeugten Wechselspannungen;

Fig. 9        die Frequenzvariation der von der Therapievorrichtung erzeugten Wechselspannung und

Fig. 10       die Variation der Maximalamplitude der von der Therapievorrichtung erzeugten Wechselspannung.

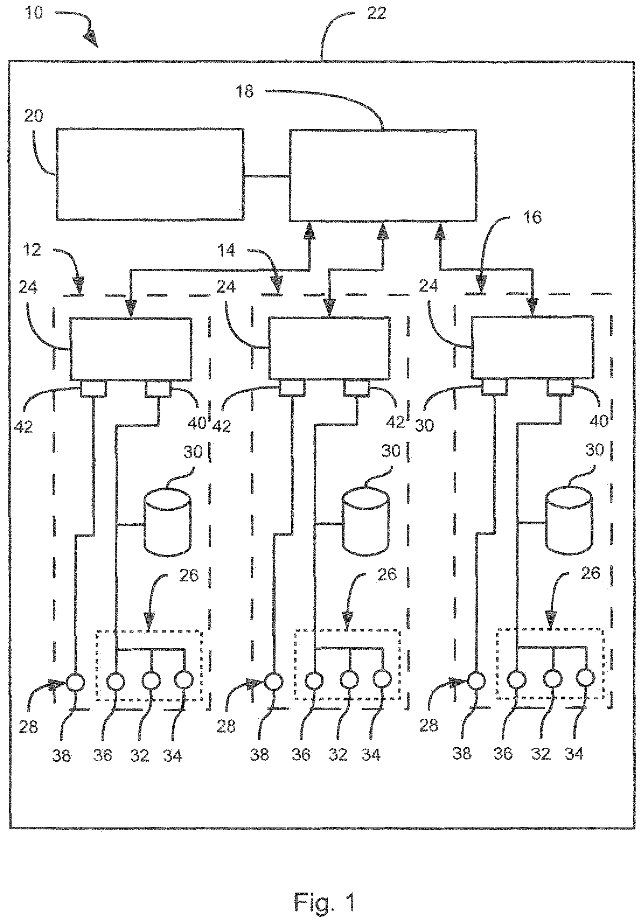

Bezug nehmend auf Fig. 1 hat eine Therapievorrichtung 10 eine erste Frequenzgeneratoreinheit 12, eine zweite Frequenzgeneratoreinheit 14, eine dritte Frequenzgeneratoreinheit 16, eine Steuereinheit 18 und einen Energiespeicher 20. Die genannten Elemente der Therapievorrichtung 10 sind in einem Gehäuse 22 angeordnet. Die drei Frequenzgeneratoreinheiten 12, 14 und 16 sind gegeneinander elektrisch isoliert und jeweils mit der Steuereinheit 18 verbunden.

Stellvertretend für die drei Frequenzgeneratoreinheiten 12, 14 und 16 soll die erste Frequenzgeneratoreinheit 12 beschrieben werden. Die erste Frequenzgeneratoreinheit 12 hat einen Frequenzgenerator 24, einen ersten Elektrodenausgang 26, einen zweiten Elektrodenausgang 28 sowie einen Behälter 30. Der erste Elektrodenausgang 26 hat einen Handelektrodenanschluss 32, einen Abgreifelektrodenanschluss 34 und einen Leiteranschluss 36. Der zweite Elektrodenausgang hat einen Neutralanschluss 38. An die Anschlüsse 32, 34, 36 und 38 kann jeweils eine Elektrode 39 angeschlossen werden. Der Leiter-Anschluss 36 und der Neutralanschluss 38 bilden einen BNC-Anschluss, wobei der Neutralanschluss 38 der Außenleiter des BNC-Anschlusses ist.

Der Frequenzgenerator 24 hat einen Frequenzausgang 40, an dem die von dem Frequenzgenerator 24 erzeugte Wechselspannung anliegt, und einen Neutratausgang 42, der mit dem Frequenzausgang 40 die Pole des Frequenzgenerators 24 bildet. Der Frequenzausgang 40 ist mit einem Behälter 30 und dem ersten Elektrodenausgang 26 verbunden. Demnach ist der Frequenzausgang 40 mit dem Handelektrodenanschluss 32, dem Abgreifelektrodenanschluss 34 und dem BNC-Anschluss 36 verbunden. Der Neutralausgang 42 ist mit dem zweiten Elektrodenausgang 28 und damit auch mit dem Neutralanschluss 38 verbunden.

Der Behälter 30 ist aus Metall hergestellt und kann mittels eines Deckels 43 verschlossen werden. Der Behälter 30 ist in dem Gehäuse 22 zweckmäßigerweise auswechselbar angeordnet. Nach Abnahme des Deckels 43 kann eine Probe in den Behälter 30 gestellt werden. An den Handelektrodenanschluss 32 kann beispielsweise eine Elektrode 39 angeschlossen werden, die von dem Patienten in der Hand gehalten werden kann. Allerdings sind auch andere Elektroden denkbar.

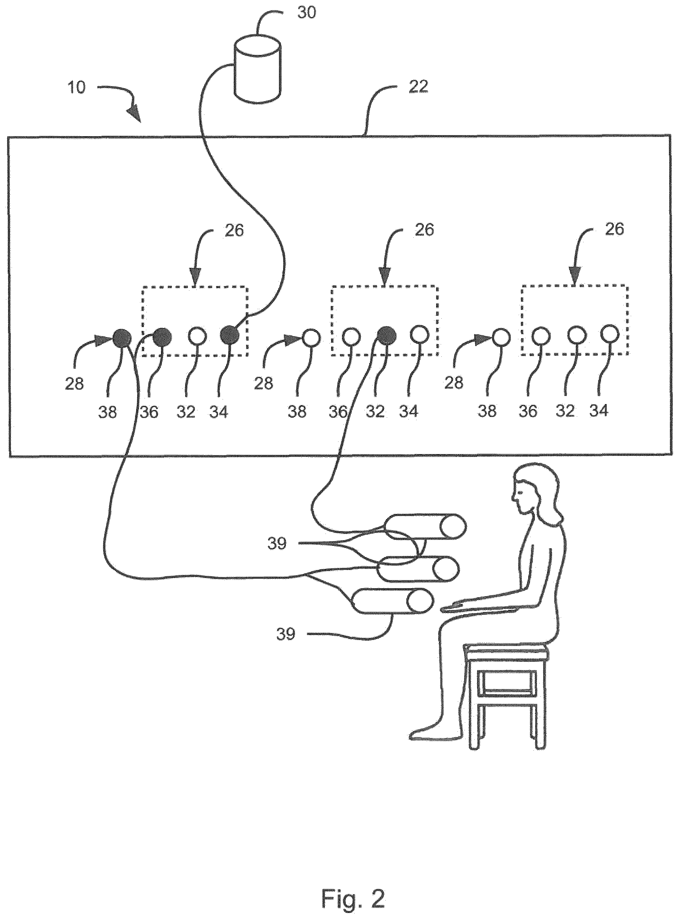

Wie in Fig. 2 zu erkennen ist, berührt ein Patient drei Elektroden 39, die als metallisches Rohr ausgeführt sind. Zwei der Elektroden 39 sind über ein BNC-Kabel mit dem BNC-Anschluss verbunden, das heißt, eine der beiden Elektroden 39 ist mit dem Leiteranschluss 36 und die andere der beiden Elektroden ist mit dem Neutralanschluss 38 verbunden. Der Leiteranschluss 36 ist mit dem Innenleiter des BNC-Kabels verbunden, während der Neutralanschluss 38 mit dem Außenleiter des BNC-Kabels verbunden ist. Die dritte gezeigte Elektrode 39 ist mit dem Handelektrodenanschluss 32 einer zweiten Frequenzgeneratoreinheit 12 verbunden. Die an den Elektroden 39 anliegende Spannung kann sich in ihrer Art, Amplitude und Phase unterscheiden.

An dem Abgreifelektrodenanschluss 34 kann beispielsweise ein weiterer Behälter 30 zur Aufnahme von Proben angeschlossen werden, wie dies in Fig. 2 ersichtlich ist.

Der Energiespeicher 20, der in dieser Ausführungsform ein wiederaufladbarer Akkumulator ist, ist mit der Steuereinheit 18 verbunden und versorgt diese mit elektrischer Energie. Die Steuereinheit 18 ist wiederum mit jeder der Frequenzgeneratoreinheiten 12, 14 und 16 verbunden und steuert jeweils die erste Frequenzgeneratoreinheit 12, die zweite Frequenzgeneratoreinheit 14 und die dritte Frequenzgeneratoreinheit 16. Ferner werden die Frequenzgeneratoreinheiten 12, 14 und 16 über die Steuereinheit 18 mit elektrischer Energie versorgt.

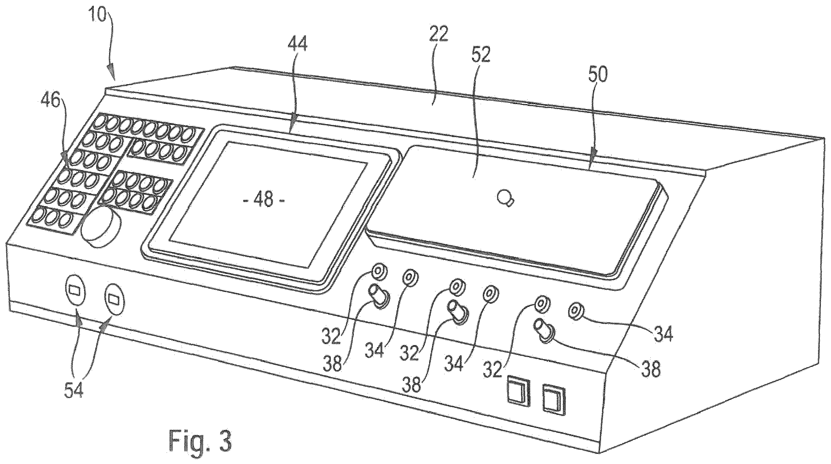

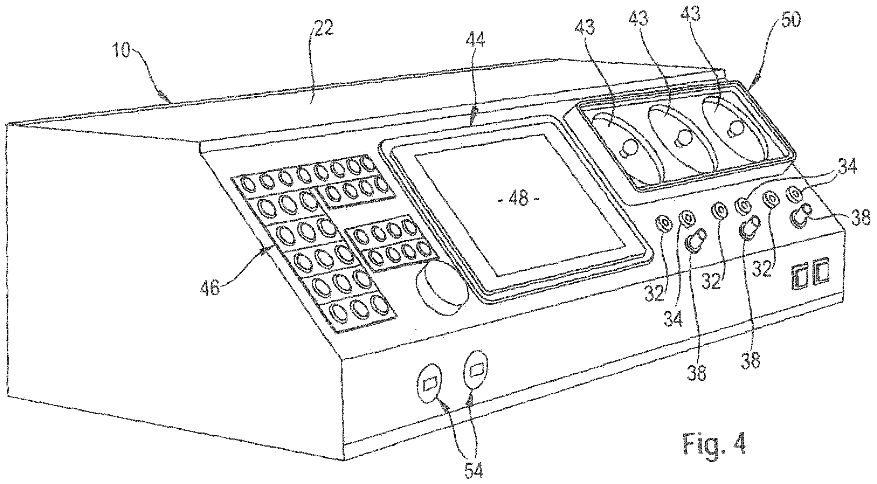

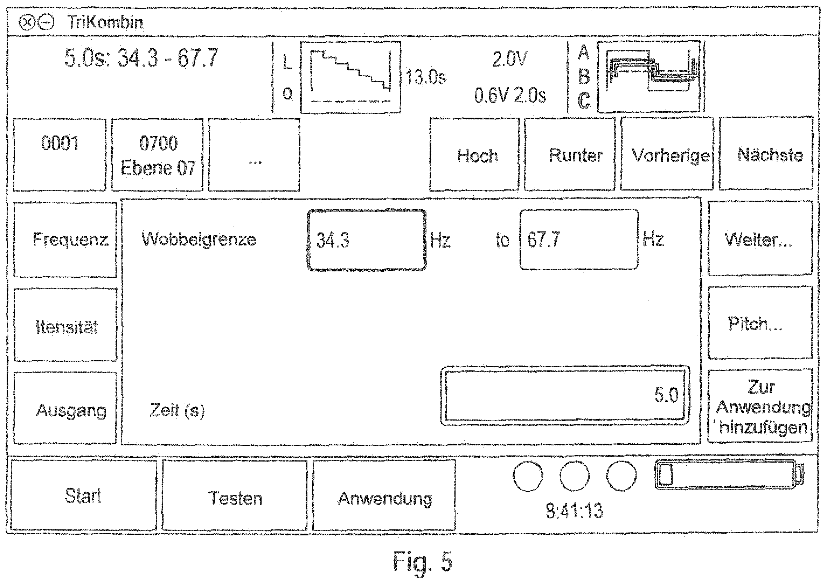

Wie aus Fig. 3 und 4 sichtbar ist, hat die Therapievorrichtung 10 ferner eine Anzeigeeinrichtung 44 und eine Eingabeeinrichtung 46. Die Anzeigeeinrichtung 44 umfasst ein Display 48, das in Fig. 5 vergrößert dargestellt ist. Auf dem Display 48 werden die Frequenz, die Spannung und die Art der Wechselspannung angegeben, die von den drei Frequenzgeneratoreinheiten 12, 14, und 16 ausgegeben wird. Ferner wird auch die Phasenbeziehung der Wechselspannung der von den drei Frequenzgeneratoreinheiten 12, 14 und 16 ausgegebenen Wechselspannungen graphisch dargestellt. Der Steuerungscomputer kann auch eine Wobbelung der Frequenz steuern, die auch auf dem Display 48 angezeigt wird. Ferner wird auch der Ladezustand des Energiespeichers 20 auf dem Display 48 angezeigt. Die Einstellung erfolgt über Knöpfe der Eingabeeinrichtung 46. Die Tasten der Eingabeeinrichtung 46 können sowohl eine Eingabe ermöglichen als auch durch das auf dem Display 48 angezeigte Menü führen. Alternativ oder zusätzlich kann das Display 48 ein Touchscreen sein, so dass eine Steuerung der Therapievorrichtung 10 auch mit Hilfe des Displays 48 möglich ist.

Die Therapievorrichtung 10 hat auch einen Behälterzugang 50, in welchem die Behälter 30 der drei Frequenzgeneratoreinheiten 12, 14 und 16 angeordnet sind. Der Behälterzugang 50 kann durch eine Klappe 52 geschlossen werden. Die Therapievorrichtung hat außerdem einen Ladegerätanschluss 54, an welchem ein nicht gezeigtes Ladegerät angeschlossen werden kann, mittels welchem der Energiespeicher 20 geladen werden kann.

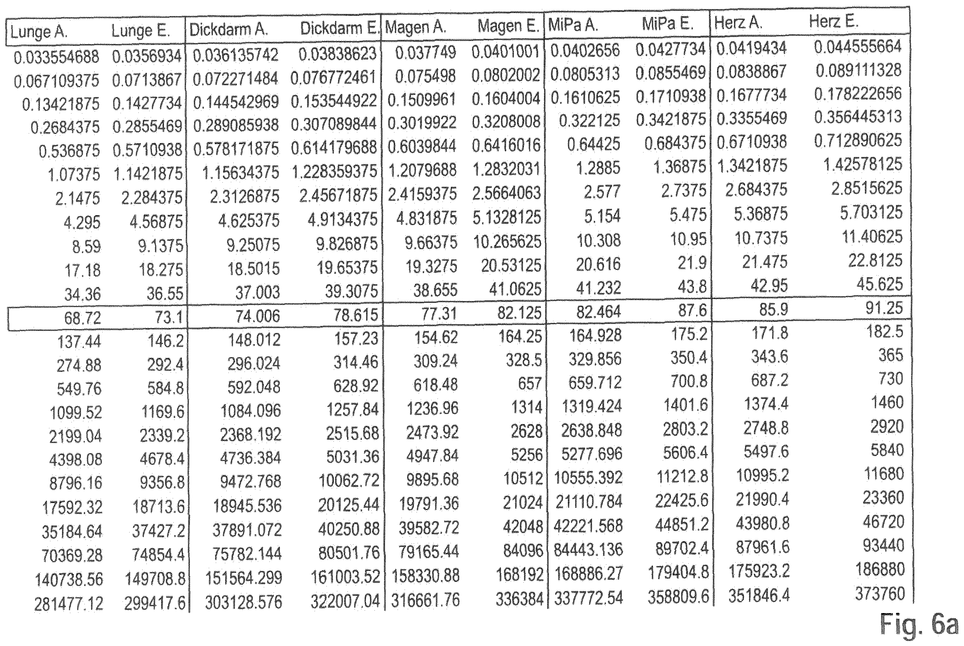

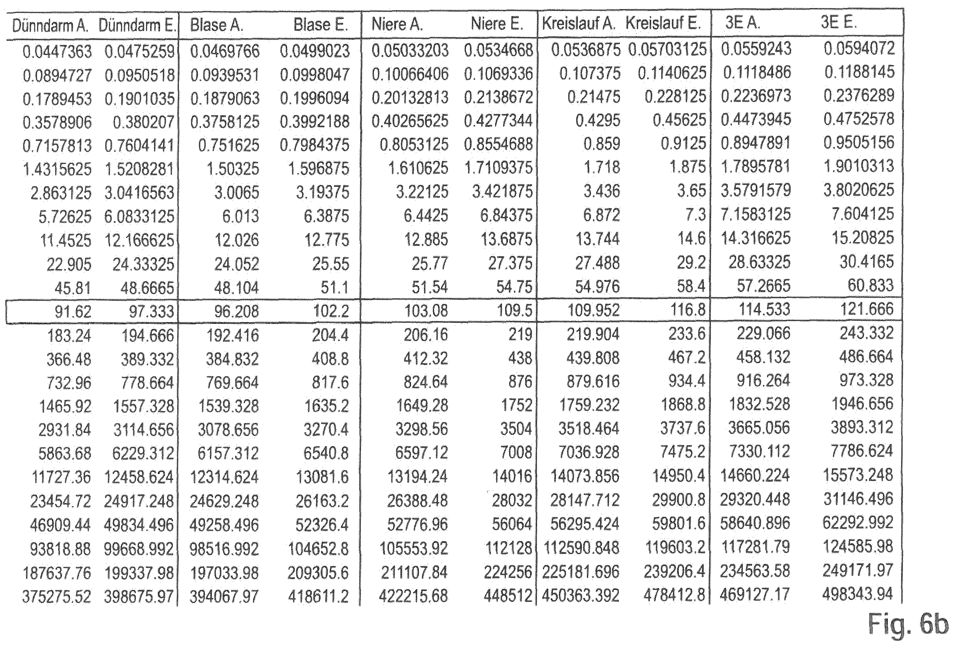

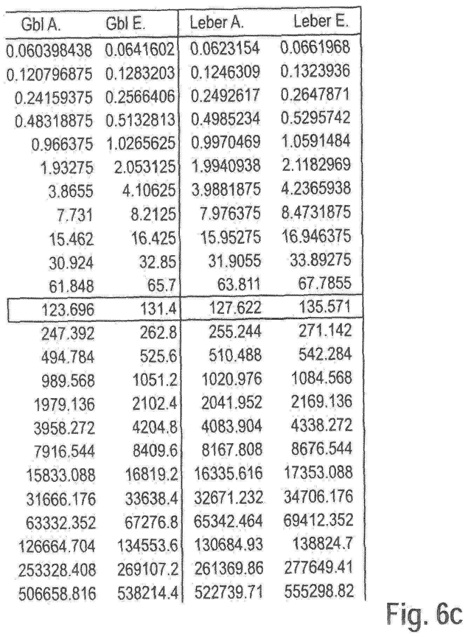

Fig. 6 zeigt eine Tabelle mit Frequenzen, die sich als besonders geeignet für die Behandlung mit elektromagnetischen Schwingungen erwiesen haben. Jede Spalte ist einem Meridian zugeordnet, der wiederum einem Organ zugeordnet ist. Jede Spalte ist ferner in zwei Unterspalten eingeteilt, wobei die linke Unterspalte die Frequenzen angibt, die mit dem Anfang des entsprechenden Meridians verknüpft sind, während die rechte Teilspalte die Frequenzen angibt, die mit dem Ende des jeweiligen Meridians verknüpft sind. Die jeweiligen Anfangs- und Endfrequenzen eines Organs sind mit den jeweiligen Anfangs- und Endfrequenzen eines anderen Organs über ein besonderes Verhältnis verknüpft. Dieses Verhältnis entspricht dem Frequenzverhältnis der Töne einer Tonleiter einer Oktave. Die Frequenzen der Lunge entsprechen dabei dem Grundton einer Tonleiter. Zur Beeinflussung der jeweiligen Organe wird die entsprechende Frequenz an der ersten Frequenzgeneratoreinheit ausgegeben.

Die Zeilen sind jeweils ein $2^Z$-faches der Grundfrequenz, die hervorgehoben ist. Das heißt, eine Frequenz kann durch Multiplikation mit 2, 4, 8 usw. oder mit 1/2, 1/4, 1/8 usw. erreicht werden. Bevorzugt gibt die erste Frequenzgeneratoreinheit 12 eine Wechselspannung mit der hervorgehobenen Frequenz aus, während die zweite 14 und dritte Frequenzgeneratoreinheit 16 jeweils eine Wechselspannung mit einer Frequenz ausgibt, die oberhalb oder unterhalb der Frequenz der ersten Frequenzgeneratoreinheit in einer Spalte angegeben ist. Alternativ können die Frequenzgeneratoreinheiten 12, 14 und 16 jeweils die gleiche Frequenz ausgeben.

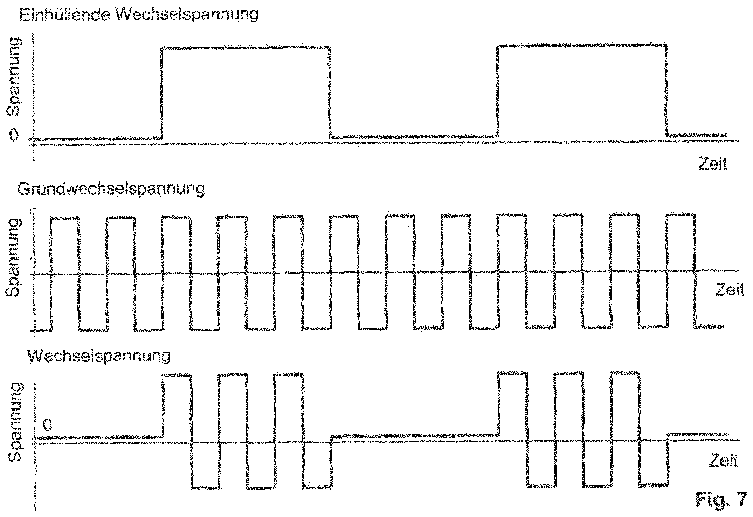

Der Verlauf der Wechselspannung, so wie er am ersten Elektrodenausgang 26 der ersten 12, zweiten 14 oder dritten Frequenzgeneratoreinheit 16 mit Hilfe eines Oszilloskops gemessen werden kann, ist in Fig. 7 unterster Graph dargestellt. Die Wechselspannung wird zwischen einer unteren Amplitudenschwelle und einer oberen Amplitudenschwelle moduliert. Die untere Amplitudenschwelle beträgt 0 V, während die obere Amplitudenschwelle 16 V beträgt. Bei einem Wert der unteren Amplitudenschwelle von 0 V liegt keine Spannung am ersten Elektrodenausgang 26 an. Die Wechselspannung wird periodisch moduliert.

Die Wechselspannung ist das Produkt aus einer einhüllenden Wechselspannung (oberster Graph) und einer Grundwechselspannung (mittlerer Graph). Die einhüllende Wechselspannung ist eine Rechteckspannung, die Werte zwischen 0 V und 1 V annimmt, so dass die Maximalamplitude 1 V ist. Die Grundwechsefspannung ist ebenfalls eine Rechteckspannung, die Werte zwischen -8 V und +8 V annimmt, so dass deren Maximalamplitude 16 V beträgt. Die Frequenz der Grundwechselspannung beträgt das 6-fache der Frequenz der einhüllenden Wechselspannung.

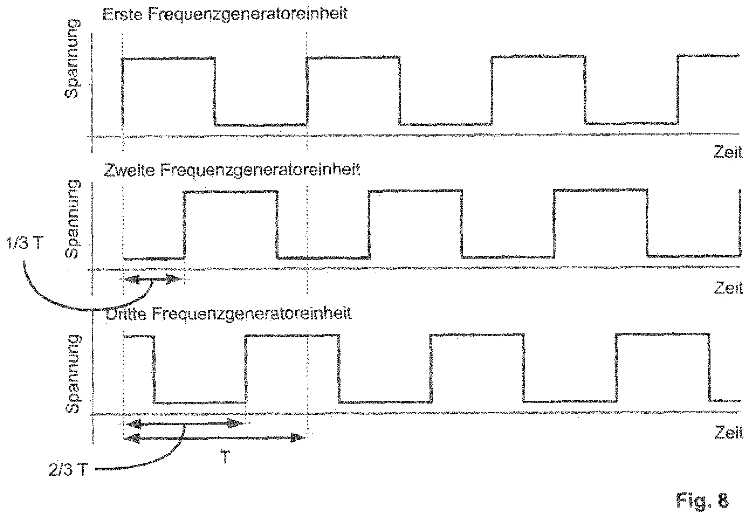

Die Phasenverschiebung der Wechselspannung zwischen der ersten 12, zweiten 14 und dritten Frequenzgeneratoreinheit 16 beträgt 120° oder ein Drittel der Periodendauer T. Dies ist in Fig. 8 dargestellt, wobei die Wechselspannung der ersten Frequenzgeneratoreinheit 12 in dem obersten Graph, der zweiten Frequenzgeneratoreinheit 14 in dem mittleren Graph und der dritten Frequenzgeneratoreinheit 16 in dem untersten Graph dargestellt ist. Die Frequenzgeneratoreinheiten 12, 14 und 16 geben jeweils eine Wechselspannung in Form einer Rechteckspannung aus. Die Amplituden der jeweiligen Rechteckspannungen sind gleich. Die Wechselspannung der ersten Frequenzgeneratoreinheit 12 hat eine erste Phase, die im vorliegenden Fall 0° ist. Die zweite Phase der Wechselspannung der zweiten Frequenzgeneratoreinheit 14 beträgt 120°. Die dritte Phase der Wechselspannung der dritten Frequenzgeneratoreinheit 16 beträgt 240°.

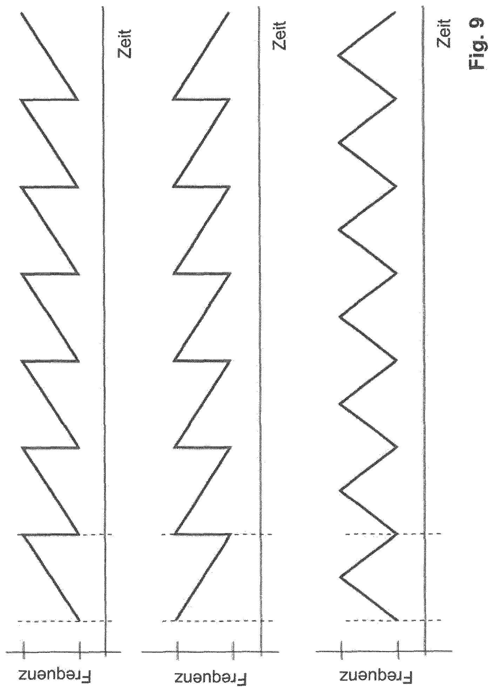

Die oberen beiden Graphen von Fig. 9 zeigen wie die Frequenz der Wechselspannung, die von der ersten 12, zweiten 14 und/oder dritten Frequenzgeneratoreinheit 16 ausgegeben wird, varriiert wird. Die Frequenz steigt gemäß der im obersten Graph gezeigten Ausfürhrungsform linear von unteren Grenzfrequenz zu einer oberen Grenzfrequenz an. Anschließend springt die Frequenz wieder auf die untere Grenzfrequenz zurück. Bei der in dem mittleren Graph gezeigten Ausführungsform fällt die Frequenz der Wechselspannung linear von der oberen Grenzfrequenz zu der unteren Grenzfrequenz ab. Anschließend springt die Frequenz wieder auf die obere Grenzfrequenz. Diese Ausführungsformen werden auch als einseitiges Wobbeln bezeichnet. Die im untersten Graphen gezeigte Frequenzvariation ist bei der Therapievorrichtung 10 nicht vorgesehen, bei welcher sowohl ein Frequenzanstieg als auch ein Frequenzabfall verwendet wird. Allerdings könnte eine solche Ausgestaltung prinzipiell auch bei der Therapievorrichtung 10 realisiert werden.

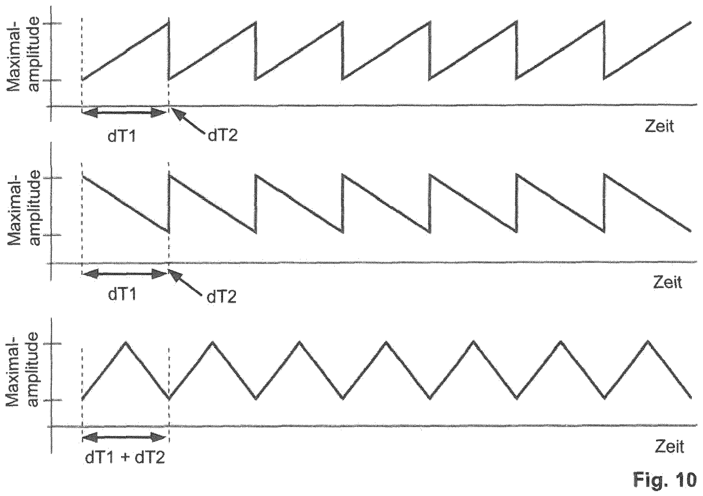

Der Verlauf der Variation der Maximalamplitude gemäß dem Sweep ist in Fig. 10 dargestellt. Der jeweils minmale Wert in dem obersten und unteresten Graph entspricht der Startamplitude und der jeweils maximale Wert entspricht der Zielamplitude. Dies entspricht einer linearen Erhöhung der Maximalamplitude. Der mittlere Graph von Fig. 10 zeigt eine alternative Ausführungsform, bei der der maximale Wert der Maximalamplitude die Startamplitude und der minimale Wert die Zielamplitude ist. Dies entspricht einem linearen Abfall der Maximalamplitude. Die erste Zeitraum dT1 ist in dem obersten und unteresten Graph die Zeit, die während des Anstiegs von der Startamplitude zu der Zielamplitude vergeht. Der zweite Zeitraum dT2 ist die Zeit, die während des Abfalls von Zielamplitude zu der Startamplitude vergeht. Im obersten Graph ist der zweite Zeitraum dT2 1 ms, so dass sich der Abfall bildlich wie ein Sprung darstellt. In dem untersten Graph von Fig. 10 ist der erste Zeitraum dT1 gleich groß wie der zweite Zeitraum dT2 dargestellt. Dies wurde jedoch nur aus Gründer der Darstellungs derart gewählt; tatsächlich ist der zweite Zeitraum dT2 kleiner als der erste Zeitraum dT1. Im mittleren Graph ist der erste Zeitraum dT1 die Zeit, die zwischen dem Abfall von der Startamplitude zu der Zielamplitude vergeht. Der zweite Zeitraum dT2 ist die Zeit, die während des Anstiegs von Zielamplitude zu der Startamplitude vergeht. Im mittleren Graph ist der zweite Zeitraum dT2 1 ms, so dass sich der Anstieg bildlich wie ein Sprung darstellt.

**Bezugszeichenliste**

10   Therapievorrichtung
12   erste Frequenzgeneratoreinheit
14   zweite Frequenzgeneratoreinheit
16   dritte Frequenzgeneratoreinheit
18   Steuereinheit
20   Energiespeicher
22   Gehäuse
24   Frequenzgenerator
26   erster Elektrodenausgang
28   zweiter Elektrodenausgang
30   Behälter
32   Handelektrodenanschluss
34   Abgreifelektrodenanschluss
36   BNC-Anschluss
38   Neutralanschluss
39   Elektrode
40   Frequenzausgang
42   Neutralausgang
43   Deckel
44   Anzeigeeinrichtung
46   Eingabeeinrichtung
48   Display
50   Behälterzugang
52   Klappe
54   Ladegerätanschluss

**Patentansprüche**

1.  Therapievorrichtung zur Behandlung von Patienten mit Hilfe von elektromagnetischen Schwingungen, umfassend:

    wenigstens eine Frequenzgeneratoreinheit (12, 14, 16) zur Erzeugung von Wechselspannung und
    eine Steuereinheit (18), welche die Frequenzgeneratoreinheit (12, 14, 16) steuert;
    wobei die Frequenzgeneratoreinheit (12, 14, 16) ausgebildet ist, die Wechselspannung mit sich zeitlich ändernder Maximalamplitude zu erzeugen, wobei die Maximalamplitude die Differenz zwischen maximaler Amplitude und minimaler Amplitude einer Periode der Wechselspannung ist;
    wobei die Steuereinheit (18) die Maximalamplitude wiederholt zwischen einer unteren Amplitudenschwelle und einer oberen Amplitudenschwelle variiert;
    **dadurch gekennzeichnet, dass** die Steuereinheit (18) die Frequenzgeneratoreinheit (12, 14, 16) derart steuert, dass die Maximalamplitude sich über einen ersten Zeitraum von einer Startamplitude zu einer Zielamplitude ändert und anschließend über einen zweiten Zeitraum auf die Startamplitude zurückkehrt, wobei der erste Zeitraum mindestens 10-fach größer als der zweite Zeitraum ist.

2.  Therapievorrichtung nach Anspruch 1, **dadurch gekennzeichnet, dass** die Maximalamplitude periodisch variiert wird.

3.  Therapievorrichtung nach Anspruch 1 oder 2, **dadurch gekennzeichnet, dass** die untere Amplitudenschwelle und/oder die obere Amplitudenschwelle jeweils innerhalb einer Periode der Wechselspannung zeitlich konstant sind.

**4.** Therapievorrichtung nach einem der Ansprüche 1 bis 3, **dadurch gekennzeichnet, dass** das Frequenzverhältnis der Frequenz der Wechselspannung zu der Frequenz der Variation der Maximalamplitude eine ganzzahlige gerade Zahl ist.

**5.** Therapievorrichtung nach Anspruch 4, **dadurch gekennzeichnet, dass** das Frequenzverhältnis zwischen 2 und 65000, insbesondere zwischen 2 und 256, beträgt.

**6.** Therapievorrichtung nach einem der Ansprüche 1 bis 5, **dadurch gekennzeichnet, dass** die Wechselspannung eine Rechteckspannung ist.

**7.** Therapievorrichtung nach einem der Ansprüche 1 bis 6, **dadurch gekennzeichnet, dass** die Frequenzgeneratoreinheit (12, 14, 16) ausgebildet ist, die Wechselspannung mit sich zeitlich ändernder Frequenz zu erzeugen, wobei die Steuereinheit (18) die Frequenz der von der Frequenzgeneratoreinheit (12, 14, 16) ausgegebenen Wechselspannung gemäß einer stetigen, insbesondere linearen, Funktion von einer unteren Grenzfrequenz zu einer oberen Grenzfrequenz oder von der oberen Grenzfrequenz zu der unteren Grenzfrequenz variiert.

**8.** Therapievorrichtung nach Anspruch 7, **dadurch gekennzeichnet, dass** die Steuereinheit (18) die Frequenz der von der Frequenzgeneratoreinheit (12, 14, 16) ausgegebenen Wechselspannung periodisch zwischen der unteren Grenzfrequenz und der oberen Grenzfrequenz variiert.

**9.** Therapievorrichtung nach einem der Ansprüche 1 bis 8, **gekennzeichnet durch** eine zweite Frequenzgeneratoreinheit (14) zur Erzeugung von Wechselspannung und/oder eine dritte Frequenzgeneratoreinheit (16) zur Erzeugung von Wechselspannung, wobei die Steuereinheit (18) die zweite Frequenzgeneratoreinheit (14) und/oder die dritte Frequenzgeneratoreinheit (16) steuert, wobei vorzugsweise die zweite Frequenzgeneratoreinheit (14) und die dritte Frequenzgeneratoreinheit (16) gegeneinander und/oder gegenüber der ersten Frequenzgeneratoreinheit (12) galvanisch getrennt sind.

**10.** Therapievorrichtung nach einem der Ansprüche 1 bis 9, umfassend:

eine erste Frequenzgeneratoreinheit (12) zur Erzeugung von Wechselspannung mit einer ersten Phase;

eine zweite Frequenzgeneratoreinheit (14) zur Erzeugung von Wechselspannung mit einer zweiten Phase und
eine Steuereinheit (18), welche die erste Frequenzgeneratoreinheit (12) und die zweite Frequenzgeneratoreinheit (14) steuert, wobei die Steuereinheit (18) die Phasenverschiebung zwischen der ersten Phase und der zweiten Phase derart regelt, dass die Phasenverschiebung zwischen 110° und 130°, insbesondere 120°, beträgt.

**11.** Therapievorrichtung nach Anspruch 10, **gekennzeichnet durch** eine dritte Frequenzgeneratoreinheit (16) zur Erzeugung von Wechselspannung mit einer dritten Phase, wobei die Steuereinheit (18) die Phasenverschiebung zwischen der zweiten Phase und der dritten Phase derart regelt, dass sie zwischen 110° und 130°, insbesondere 120°, beträgt.

**12.** Therapievorrichtung nach Anspruch 11, **dadurch gekennzeichnet, dass** die Steuereinheit (18) die Frequenzgeneratoreinheiten (12, 14, 16) derart regelt, dass die Maximalamplitude der von der ersten Frequenzgeneratoreinheit (12) erzeugten Wechselspannung größer als die Maximalamplitude der von der zweiten Frequenzgeneratoreinheit (14) erzeugten Wechselspannung ist und dass vorzugsweise die Maximalamplitude der von der zweiten Frequenzgeneratoreinheit (14) erzeugten Wechselspannung größer als die Maximalamplitude der von der dritten Frequenzgeneratoreinheit (16) erzeugten Wechselspannung ist.

**13.** Therapievorrichtung nach einem der Ansprüche 11 bis 12, **dadurch gekennzeichnet, dass** die zweite Frequenzgeneratoreinheit (14) und die dritte Frequenzgeneratoreinheit (16) gegeneinander und/oder gegenüber der ersten Frequenzgeneratoreinheit (12) galvanisch getrennt sind.

**14.** Therapievorrichtung nach einem der Ansprüche 11 bis 13, **dadurch gekennzeichnet, dass** die erste Frequenzgeneratoreinheit (12) einen ersten Frequenzausgang (40), an dem die erzeugte Wechselspannung anliegt, und einen ersten Neutralausgang (42) aufweist, und/oder die zweite Frequenzgeneratoreinheit (14) einen zweiten Frequenzausgang (40), an dem die erzeugte Wechselspannung anliegt, und einen zweiten Neutralausgang (42) aufweist, und/oder die dritte Frequenzgeneratoreinheit (16) einen dritten Frequenzausgang (40), an dem die erzeugte Wechselspannung anliegt, und einen dritten Neutralausgang (42) aufweist.

**15.** Therapievorrichtung nach einem der Ansprüche 1 bis 14, **dadurch gekennzeichnet, dass** der erste Zeitraum mindestens 100-fach größer als der zweite Zeitraum ist.

## Claims

1. Therapy device for the treatment of patients with the aid of electromagnetic oscillations comprising:

   at least one frequency generator unit (12, 14, 16) for generating alternating voltage and
   a control unit (18) which controls said frequency generator unit (12, 14, 16);
   wherein the frequency generator unit (12, 14, 16) is configured to generate the alternating voltage having a time-varying maximum amplitude, wherein the maximum amplitude is the difference between maximum amplitude and minimum amplitude of a period of the alternating voltage;
   wherein the control unit (18) repeatedly varies the maximum amplitude between a lower amplitude threshold and an upper amplitude threshold;
   **characterized in that** the control unit (18) controls the frequency generator unit (12, 14, 16) in such a way that the maximum amplitude changes over a first period of time from a start amplitude to a target amplitude and subsequently returns to the start amplitude over a second period of time, wherein the first period is at least 10 times longer than the second period.

2. Therapy device according to claim 1, **characterized in that** the maximum amplitude is varied periodically.

3. Therapy device according to claim 1 or 2, **characterised in that** the lower amplitude threshold and/or the upper amplitude threshold are each constant in time within one period of the alternating voltage.

4. Therapy device according to any one of claims 1 to 3, **characterized in that** the frequency ratio of the frequency of the alternating voltage to the frequency of the variation of the maximum amplitude is an integer even number.

5. Therapy device according to claim 4, **characterized in that** the frequency ratio is between 2 and 65000, in particular between 2 and 256.

6. Therapy device according to any one of claims 1 to 5, **characterized in that** the alternating voltage is a rectangular voltage.

7. Therapy device according to any one of claims 1 to 6, **characterized in that** the frequency generator unit (12, 14, 16) is configured to generate alternating voltage having a time-varying frequency, wherein the control unit (18) varies the frequency of the alternating voltage output by the frequency generator unit (12, 14, 16) according to a continuous, especially linear, function from a lower cut-off frequency to an upper cut-off frequency or from the upper cut-off frequency to the lower cut-off frequency.

8. Therapy device according to claim 7, **characterized in that** the control unit (18) periodically varies the frequency of the alternating voltage output by the frequency generator unit (12, 14, 16) between the lower cut-off frequency and the upper cut-off frequency.

9. Therapy device according to any one of claims 1 to 8, **characterized by**
   a second frequency generator unit (14) for generating alternating voltage and/or
   a third frequency generator unit (16) for generating alternating voltage, wherein the control unit (18) controls the second frequency generator unit (14) and/or the third frequency generator unit (16),
   wherein preferably the second frequency generator unit (14) and the third frequency generator unit (16) are opposed to one another and/or are galvanically isolated with respect to the first frequency generator unit (12).

10. A therapy device according to any one of claims 1 to 9 comprising:

    a first frequency generator unit (12) for generating alternating voltage having a first phase;
    a second frequency generator unit (14) for generating alternating voltage having a second phase and
    a control unit (18) which controls the first a frequency generator unit (12) and the second frequency generator unit (14),
    the control unit (18) controlling the phase shift between the first phase and the second phase in such a way that the phase shift is between 110° and 130°, in particular 120°.

11. Therapy device according to claim 10, **characterized by** a third frequency generator unit (16) for generating alternating voltage having a third phase, the control unit (18) regulating the phase shift between the second phase and the third phase in such a way that it is between 110° and 130°, in particular 120°.

12. Therapy device according to claim 11, **characterized in that** the control unit (18) controls the frequency generator units (12, 14, 16) in such a way that the maximum amplitude of the alternating voltage generated by the first frequency generator unit (12) is greater than the maximum amplitude of the alternating voltage generated by the second frequency generator unit (14), and **in that** preferably the maximum amplitude of the alternating voltage generated by the second frequency generator unit (14) is greater than the maximum amplitude of the alternating voltage generated by the third frequency generator unit (16).

13. Therapy device according to claim 11 or 12, **characterized in that** the second frequency generator unit (14) and the third frequency generator unit (16) are galvanically isolated from one another and/or from the first frequency generator unit (12).

14. Therapy device according to any one of claims 11 to 13, **characterized in that**
the first frequency generator unit (12) has a first frequency output (40) at which the generated alternating voltage is applied, and a first neutral output (42), and/or
the second frequency generator unit (14) has a second frequency output (40), at which the generated alternating voltage is applied, and a second neutral output (42), and/or
the third frequency generator unit (16) has a third frequency output (40), at which the generated alternating voltage is applied, and a third neutral output (42).

15. Therapy device according to any one of claims 1 to 14, **characterized in that** the first period is at least 100 times greater than the second period.

## Revendications

1. Dispositif de thérapie pour le traitement de patients à l'aide d'oscillations électromagnétiques, incluant:

au moins une unité génératrice de fréquence (12, 14, 16) pour engendrer une tension alternative et
une unité de commande (18), qui commande l'unité génératrice de fréquence (12, 14, 16);
dans lequel l'unité génératrice de fréquence (12, 14, 16) est réalisée pour engendrer la tension alternative avec une amplitude maximum variable au cours du temps, ladite amplitude maximum étant la différence entre l'amplitude maximale et l'amplitude minimale d'une période de la tension alternative;
dans lequel l'unité de commande (18) varie l'amplitude maximum de façon répétée entre un seuil d'amplitude inférieur et un seuil d'amplitude supérieur;
**caractérisé en ce que** l'unité de commande (18) commande l'unité génératrice de fréquence (12, 14, 16) de telle façon que l'amplitude maximum varie sur une première durée temporelle depuis une amplitude de départ jusqu'à une amplitude cible et retourne ensuite à l'amplitude de départ sur une deuxième durée temporelle, la première durée temporelle étant au moins 10 fois plus longue que la deuxième durée temporelle.

2. Dispositif de thérapie selon la revendication 1, **caractérisé en ce que** l'amplitude maximum varie périodiquement.

3. Dispositif de thérapie selon la revendication 1 ou 2, **caractérisé en ce que** le seuil d'amplitude inférieur et/ou le seuil d'amplitude supérieur sont constants respectivement à l'intérieur d'une période de la tension alternative.

4. Dispositif de thérapie selon l'une des revendications 1 à 3, **caractérisé en ce que** le rapport de la fréquence de la tension alternative sur la fréquence de la variation de l'amplitude maximum est un nombre entier pair.

5. Dispositif de thérapie selon la revendication 4, **caractérisé en ce que** le rapport de fréquence est entre 2 et 65 000, en particulier entre 2 et 256.

6. Dispositif de thérapie selon l'une des revendications 1 à 5, **caractérisé en ce que** la tension alternative est une tension rectangulaire.

7. Dispositif de thérapie selon l'une des revendications 1 à 6, **caractérisé en ce que**
l'unité génératrice de fréquence (12, 14, 16) est réalisée pour engendrer la tension alternative avec une fréquence variable au cours du temps,
dans lequel l'unité de commande (18) fait varier la fréquence de la tension alternative délivrée par l'unité génératrice de fréquence (12, 14, 16) selon une fonction constante, en particulier linéaire, depuis une fréquence limite inférieure jusqu'à une fréquence limite supérieure ou depuis la fréquence limite supérieure jusqu'à la fréquence limite inférieure.

8. Dispositif de thérapie selon la revendication 7, **caractérisé en ce que** l'unité de commande (18) fait varier la fréquence de la tension alternative délivrée par l'unité génératrice de fréquence (12, 14, 16) périodiquement entre la fréquence limite inférieure et la fréquence limite supérieure.

9. Dispositif de thérapie selon l'une des revendications 1 à 8, **caractérisé par**:

une deuxième unité génératrice de fréquence (14) pour engendrer une tension alternative et/ou

une troisième unité génératrice de fréquence (16) pour engendrer une tension alternative, dans lequel l'unité de commande (18) commande la deuxième unité génératrice de fréquence (14) et/ou la troisième unité génératrice de fréquence (16),

dans lequel la deuxième unité génératrice de fréquence (14) et la troisième unité génératrice de fréquence (16) sont de préférence séparées sur le plan galvanique l'une par rapport à l'autre et/ou par rapport à la première unité génératrice de fréquence (12).

10. Dispositif de thérapie selon l'une des revendications 1 à 9, incluant une première unité génératrice de fréquence (12) pour engendrer une tension alternative avec une première phase ;

une deuxième unité génératrice de fréquence (14) pour engendrer une tension alternative avec une deuxième phase, et

une unité de commande (18) qui commande la première unité génératrice de fréquence (12) et la deuxième unité génératrice de fréquence (14),

dans lequel l'unité de commande (18) régule le décalage de phase entre la première phase et la deuxième phase de telle façon que le décalage de phase est entre 110° et 130°, en particulier 120°.

11. Dispositif de thérapie selon la revendication 10, **caractérisé par** une troisième unité génératrice de fréquence (16) pour engendrer une tension alternative avec une troisième phase,

dans lequel l'unité de commande (18) régule le décalage de phase entre la deuxième phase et la troisième phase de telle façon que celui-ci est entre 110° et 130°, en particulier 120°.

12. Dispositif de thérapie selon la revendication 11, **caractérisé en ce que** l'unité de commande (18) régule les unités génératrices de fréquence (12, 14, 16) de telle façon que l'amplitude maximum de la tension alternative engendrée par la première unité génératrice de fréquence (12) est supérieure à l'amplitude maximum de la tension alternative engendrée par la deuxième unité génératrice de fréquence (14), et **en ce que** de préférence l'amplitude maximum de la tension alternative engendrée par la deuxième unité génératrice de fréquence (14) est supérieure à l'amplitude maximum de la tension alternative engendrée par la troisième unité génératrice de fréquence (16).

13. Dispositif de thérapie selon l'une des revendications 11 ou 12, **caractérisé en ce que** la deuxième unité génératrice de fréquence (14) et la troisième unité génératrice de fréquence (16) sont séparées sur le plan galvanique l'une par rapport à l'autre et/ou par rapport à la première unité génératrice de fréquence (12).

14. Dispositif de thérapie selon l'une des revendications 11 à 13, **caractérisé en ce que** la première unité génératrice de fréquence (12) comprend une première sortie de fréquence (40) à laquelle est appliquée la tension alternative engendrée, et une première sortie neutre (42), et/ou la deuxième unité génératrice de fréquence (14) comprend une deuxième sortie de fréquence (40) à laquelle est appliquée la tension alternative engendrée, et une deuxième sortie neutre (42), et/ou la troisième unité génératrice de fréquence (16) comprend une troisième sortie de fréquence (40) à laquelle est appliquée la tension alternative engendrée, et une troisième sortie neutre (42).

15. Dispositif de thérapie selon l'une des revendications 1 à 14, **caractérisé en ce que** la première durée temporelle est au moins 100 fois plus longue que la deuxième durée temporelle.

Fig. 1

EP 3 131 621 B1

Fig. 2

16

Fig. 3

Fig. 4

EP 3 131 621 B1

18

⊗⊖  TriKombin

| 5.0s: 34.3 - 67.7 | L 0 | 13.0s | 2.0V  0.6V 2.0s | A B C |

| 0001 | 0700 Ebene 07 | ... | | | Hoch | Runter | Vorherige | Nächste |

| Frequenz | Wobbelgrenze | 34.3 | Hz | to | 67.7 | Hz | Weiter... |

| Itensität | | | | | | | Pitch... |

| Ausgang | Zeit (s) | | | | 5.0 | | Zur Anwendung hinzufügen |

| Start | Testen | Anwendung | ○ ○ ○  8:41:13 | |

Fig. 5

| Lunge A. | Lunge E. | Dickdarm A. | Dickdarm E. | Magen A. | Magen E. | MiPa A. | MiPa E. | Herz A. | Herz E. |
|---|---|---|---|---|---|---|---|---|---|
| 0.033554688 | 0.0356934 | 0.036135742 | 0.03838623 | 0.037749 | 0.0401001 | 0.0402656 | 0.0427734 | 0.0419434 | 0.044555664 |
| 0.067109375 | 0.0713867 | 0.072271484 | 0.076772461 | 0.075498 | 0.0802002 | 0.0805313 | 0.0855469 | 0.0838867 | 0.089111328 |
| 0.13421875 | 0.1427734 | 0.144542969 | 0.153544922 | 0.1509961 | 0.1604004 | 0.1610625 | 0.1710938 | 0.1677734 | 0.178222656 |
| 0.2684375 | 0.2855469 | 0.289085938 | 0.307089844 | 0.3019922 | 0.3208008 | 0.322125 | 0.3421875 | 0.3355469 | 0.356445313 |
| 0.536875 | 0.5710938 | 0.578171875 | 0.614179688 | 0.6039844 | 0.6416016 | 0.64425 | 0.684375 | 0.6710938 | 0.712890625 |
| 1.07375 | 1.1421875 | 1.15634375 | 1.228359375 | 1.2079688 | 1.2832031 | 1.2885 | 1.36875 | 1.3421875 | 1.42578125 |
| 2.1475 | 2.284375 | 2.3126875 | 2.45671875 | 2.4159375 | 2.5664063 | 2.577 | 2.7375 | 2.684375 | 2.8515625 |
| 4.295 | 4.56875 | 4.625375 | 4.9134375 | 4.831875 | 5.1328125 | 5.154 | 5.475 | 5.36875 | 5.703125 |
| 8.59 | 9.1375 | 9.25075 | 9.826875 | 9.66375 | 10.265625 | 10.308 | 10.95 | 10.7375 | 11.40625 |
| 17.18 | 18.275 | 18.5015 | 19.65375 | 19.3275 | 20.53125 | 20.616 | 21.9 | 21.475 | 22.8125 |
| 34.36 | 36.55 | 37.003 | 39.3075 | 38.655 | 41.0625 | 41.232 | 43.8 | 42.95 | 45.625 |
| 68.72 | 73.1 | 74.006 | 78.615 | 77.31 | 82.125 | 82.464 | 87.6 | 85.9 | 91.25 |
| 137.44 | 146.2 | 148.012 | 157.23 | 154.62 | 164.25 | 164.928 | 175.2 | 171.8 | 182.5 |
| 274.88 | 292.4 | 296.024 | 314.46 | 309.24 | 328.5 | 329.856 | 350.4 | 343.6 | 365 |
| 549.76 | 584.8 | 592.048 | 628.92 | 618.48 | 657 | 659.712 | 700.8 | 687.2 | 730 |
| 1099.52 | 1169.6 | 1084.096 | 1257.84 | 1236.96 | 1314 | 1319.424 | 1401.6 | 1374.4 | 1460 |
| 2199.04 | 2339.2 | 2368.192 | 2515.68 | 2473.92 | 2628 | 2638.848 | 2803.2 | 2748.8 | 2920 |
| 4398.08 | 4678.4 | 4736.384 | 5031.36 | 4947.84 | 5256 | 5277.696 | 5606.4 | 5497.6 | 5840 |
| 8796.16 | 9356.8 | 9472.768 | 10062.72 | 9895.68 | 10512 | 10555.392 | 11212.8 | 10995.2 | 11680 |
| 17592.32 | 18713.6 | 18945.536 | 20125.44 | 19791.36 | 21024 | 21110.784 | 22425.6 | 21990.4 | 23360 |
| 35184.64 | 37427.2 | 37891.072 | 40250.88 | 39582.72 | 42048 | 42221.568 | 44851.2 | 43980.8 | 46720 |
| 70369.28 | 74854.4 | 75782.144 | 80501.76 | 79165.44 | 84096 | 84443.136 | 89702.4 | 87961.6 | 93440 |
| 140738.56 | 149708.8 | 151564.299 | 161003.52 | 158330.88 | 168192 | 168886.27 | 179404.8 | 175923.2 | 186880 |
| 281477.12 | 299417.6 | 303128.576 | 322007.04 | 316661.76 | 336384 | 337772.54 | 358809.6 | 351846.4 | 373760 |

Fig. 6a

EP 3 131 621 B1

| Dünndarm A. | Dünndarm E. | Blase A. | Blase E. | Niere A. | Niere E. | Kreislauf A. | Kreislauf E. | 3E A. | 3E E. |
|---|---|---|---|---|---|---|---|---|---|
| 0.0447363 | 0.0475259 | 0.0469766 | 0.0499023 | 0.05033203 | 0.0534668 | 0.0536875 | 0.05703125 | 0.0559243 | 0.0594072 |
| 0.0894727 | 0.0950518 | 0.0939531 | 0.0998047 | 0.10066406 | 0.1069336 | 0.107375 | 0.1140625 | 0.1118486 | 0.1188145 |
| 0.1789453 | 0.1901035 | 0.1879063 | 0.1996094 | 0.20132813 | 0.2138672 | 0.21475 | 0.228125 | 0.2236973 | 0.2376289 |
| 0.3578906 | 0.380207 | 0.3758125 | 0.3992188 | 0.40265625 | 0.4277344 | 0.4295 | 0.45625 | 0.4473945 | 0.4752578 |
| 0.7157813 | 0.7604141 | 0.751625 | 0.7984375 | 0.8053125 | 0.8554688 | 0.859 | 0.9125 | 0.8947891 | 0.9505156 |
| 1.4315625 | 1.5208281 | 1.50325 | 1.596875 | 1.610625 | 1.7109375 | 1.718 | 1.875 | 1.7895781 | 1.9010313 |
| 2.863125 | 3.0416563 | 3.0065 | 3.19375 | 3.22125 | 3.421875 | 3.436 | 3.65 | 3.5791579 | 3.8020625 |
| 5.72625 | 6.0833125 | 6.013 | 6.3875 | 6.4425 | 6.84375 | 6.872 | 7.3 | 7.1583125 | 7.604125 |
| 11.4525 | 12.166625 | 12.026 | 12.775 | 12.885 | 13.6875 | 13.744 | 14.6 | 14.316625 | 15.20825 |
| 22.905 | 24.33325 | 24.052 | 25.55 | 25.77 | 27.375 | 27.488 | 29.2 | 28.63325 | 30.4165 |
| 45.81 | 48.6665 | 48.104 | 51.1 | 51.54 | 54.75 | 54.976 | 58.4 | 57.2665 | 60.833 |
| 91.62 | 97.333 | 96.208 | 102.2 | 103.08 | 109.5 | 109.952 | 116.8 | 114.533 | 121.666 |
| 183.24 | 194.666 | 192.416 | 204.4 | 206.16 | 219 | 219.904 | 233.6 | 229.066 | 243.332 |
| 366.48 | 389.332 | 384.832 | 408.8 | 412.32 | 438 | 439.808 | 467.2 | 458.132 | 486.664 |
| 732.96 | 778.664 | 769.664 | 817.6 | 824.64 | 876 | 879.616 | 934.4 | 916.264 | 973.328 |
| 1465.92 | 1557.328 | 1539.328 | 1635.2 | 1649.28 | 1752 | 1759.232 | 1868.8 | 1832.528 | 1946.656 |
| 2931.84 | 3114.656 | 3078.656 | 3270.4 | 3298.56 | 3504 | 3518.464 | 3737.6 | 3665.056 | 3893.312 |
| 5863.68 | 6229.312 | 6157.312 | 6540.8 | 6597.12 | 7008 | 7036.928 | 7475.2 | 7330.112 | 7786.624 |
| 11727.36 | 12458.624 | 12314.624 | 13081.6 | 13194.24 | 14016 | 14073.856 | 14950.4 | 14660.224 | 15573.248 |
| 23454.72 | 24917.248 | 24629.248 | 26163.2 | 26388.48 | 28032 | 28147.712 | 29900.8 | 29320.448 | 31146.496 |
| 46909.44 | 49834.496 | 49258.496 | 52326.4 | 52776.96 | 56064 | 56295.424 | 59801.6 | 58640.896 | 62292.992 |
| 93818.88 | 99668.992 | 98516.992 | 104652.8 | 105553.92 | 112128 | 112590.848 | 119603.2 | 117281.79 | 124585.98 |
| 187637.76 | 199337.98 | 197033.98 | 209305.6 | 211107.84 | 224256 | 225181.696 | 239206.4 | 234563.58 | 249171.97 |
| 375275.52 | 398675.97 | 394067.97 | 418611.2 | 422215.68 | 448512 | 450363.392 | 478412.8 | 469127.17 | 498343.94 |

Fig. 6b

EP 3 131 621 B1

| Gbl A. | Gbl E. | Leber A. | Leber E. |
|---|---|---|---|
| 0.060398438 | 0.0641602 | 0.0623154 | 0.0661968 |
| 0.120796875 | 0.1283203 | 0.1246309 | 0.1323936 |
| 0.24159375 | 0.2566406 | 0.2492617 | 0.2647871 |
| 0.48318875 | 0.5132813 | 0.4985234 | 0.5295742 |
| 0.966375 | 1.0265625 | 0.9970469 | 1.0591484 |
| 1.93275 | 2.053125 | 1.9940938 | 2.1182969 |
| 3.8655 | 4.10625 | 3.9881875 | 4.2365938 |
| 7.731 | 8.2125 | 7.976375 | 8.4731875 |
| 15.462 | 16.425 | 15.95275 | 16.946375 |
| 30.924 | 32.85 | 31.9055 | 33.89275 |
| 61.848 | 65.7 | 63.811 | 67.7855 |
| 123.696 | 131.4 | 127.622 | 135.571 |
| 247.392 | 262.8 | 255.244 | 271.142 |
| 494.784 | 525.6 | 510.488 | 542.284 |
| 989.568 | 1051.2 | 1020.976 | 1084.568 |
| 1979.136 | 2102.4 | 2041.952 | 2169.136 |
| 3958.272 | 4204.8 | 4083.904 | 4338.272 |
| 7916.544 | 8409.6 | 8167.808 | 8676.544 |
| 15833.088 | 16819.2 | 16335.616 | 17353.088 |
| 31666.176 | 33638.4 | 32671.232 | 34706.176 |
| 63332.352 | 67276.8 | 65342.464 | 69412.352 |
| 126664.704 | 134553.6 | 130684.93 | 138824.7 |
| 253328.408 | 269107.2 | 261369.86 | 277649.41 |
| 506658.816 | 538214.4 | 522739.71 | 555298.82 |

Fig. 6c

Einhüllende Wechselspannung

Grundwechselspannung

Wechselspannung

Fig. 7

Fig. 8

Erste Frequenzgeneratoreinheit

Zweite Frequenzgeneratoreinheit

Dritte Frequenzgeneratoreinheit

Spannung

Zeit

1/3 T

2/3 T

T

Fig. 9

Fig. 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102013102275 A1
- US 2006195153 A1
- US 2007179564 A1